# EUROPEAN PATENT APPLICATION

(11) **EP 4 759 815 A1**
(43) Date of publication of application: **17.06.2026**
(21) Application number: 24853699.7
(22) Date of filing: 09.08.2024
(51) Int. Cl.: C07D 487/06, A61K 31/4985, A61K 31/519, A61P 35/00

(54) **FUSED TRICYCLIC COMPOUND, PHARMACEUTICAL COMPOSITION THEREOF, AND USE THEREOF**

(30) Priority: 11.08.2023 CN 202311012070
(71) Applicant: Haihe Biopharma Co., Ltd., Shanghai 201203 (CN)
(72) Inventor: GAO, Shanyun, Shanghai 201203 (CN); HOU, Yingjie, Shanghai 201203 (CN); LI, Jingjing, Shanghai 201203 (CN); ZHANG, Chaobo, Shanghai 201203 (CN); XU, Yanxiao, Shanghai 201203 (CN); TU, Wangyang, Shanghai 201203 (CN); YU, Bing, Shanghai 201203 (CN); ZHANG, Yixiang, Shanghai 201203 (CN); LI, Leping, Shanghai 201203 (CN)
(74) Representative: Petraz, Gilberto Luigi
(86) International application number: PCT/CN2024/111065
(87) International publication number: WO 2025/036290

(57) **Abstract**

The present invention provided a fused tricyclic compound represented by formula (I), a pharmaceutical composition thereof, and use thereof. The compound has high selectivity for PARP1, has fewer side effects than Olaparib, has high clinical application value, and can be used to prepare drugs for preventing and/or treating diseases, in particular diseases ameliorated by the inhibition of PARP1.

## Description

### CROSS-REFERENCES TO RELATED APPLICATIONS

The present application claims priority for Chinese patent application with application number 202311012070.4, filed on August 11, 2023, the entire disclosures of which are incorporated herein by reference in their entirety.

### TECHNICAL FIELD

The present invention relates to the technical field of PARP inhibitor synthesis, and in particular to a class of fused tricyclic compounds, pharmaceutical compositions thereof, and use thereof as PARP1 inhibitors in the preparation of drugs for preventing and/or treating diseases improved by inhibiting PARP1.

### BACKGROUND

Poly (ADP-ribose) polymerases (PARPs) are an emerging family of enzymes that catalyze the transfer of ADP-ribose to target proteins (poly ADP-ribosylation). At least 18 PARP family members are encoded by different genes and share homology in the conserved catalytic domain (Morales et al, Critical Reviews™ in Eukaryotic Gene Expression 24.1, 2014). PARP1 as an abundant nuclear protein can catalyze the transfer of ADP-ribose residues from NAD+ to target substrate proteins or nucleic acids, constructing a poly (ADP-ribose) (PAR) chain to be added to the downstream target proteins, a post-translational modification known as PARylation (Murai et al, Cancer research 72, 21, 2012). PARPs are important in several cellular processes including cell proliferation and cell death. The primary function of PARPs is to participate in DNA damage repair, with DNA single-strand breaks (SSBs) being the most common type of damage, which can be converted into potentially cleavage-causing and lethal DNA double-strand breaks (DSBs). PARP1 binds to damaged DNA at single-stranded DNA breaks (SSBs) and other DNA damages, an event that leads to a series of conformational changes in the structure of PARP1, which activates its catalytic function (Lord et al, Science 355, 6330, 2017).

BRCA1 and BRCA2 proteins are essential for the repair of double-stranded DNA breaks (DSBs) by a process known as homologous recombination repair (HRR), a form of DNA repair that utilizes homologous DNA sequences to direct repair at the DSB. HRR is usually a 'conserved' mechanism because it restores the original DNA sequence at the site of DNA damage. Non-conserved forms of DNA repair, such as non-homologous end joining (NHEJ), predominate when cells are HRR-deficient, whether driven by defects in BRCA1, BRCA2 or other pathway components.

PARP inhibitors play an anticancer role by blocking DNA damage repair in highly mutated cancer cells, resulting in "toxic damage" that causes cell death in cells lacking homologous recombination repair (HRR). Multiple signaling pathways for DNA repair exist in healthy cells, so inhibiting PARP alone will not be too toxic to them. However, for some tumor cells, the DNA repair pathway will be PARP1 dependent and therefore will be particularly sensitive to PARP inhibitors, as mutations in specific genes such as BRCA disrupt other DNA repair pathways. This is why ovarian and breast cancer patients carrying BRCA mutations are more likely to benefit from PARP inhibitors. PARP2 has a low intracellular content, accounting for only 5% ~ 10% of total PARP activity. Knockdown of PARP1, compared to knockdown of PARP2 (<10%) significantly reduced PARP activity (Yélamos et al, The EMBO journal 25.18, 2006). Knockdown of PARP1 blocked the PARP inhibitory activity of Olaparib and also eliminated the cell proliferation inhibitory effects of Olaparib (Murai et al, Cancer research, 2012). These data suggest that the key determinant of the antitumor effect of PARP inhibitors is the inhibition of PARP1.

It has been reported that mice require intact PARP2 in the bone marrow for survival. PARP2 deficiency results in reduced numbers of RBCs, WBCs and BM cells (Farrés et al, Blood, The Journal of the American Society of Hematology 122, 1, 2013). Knockdown of PARP2 reduced the number of T cells and RBCs, whereas knockdown of PARP1 did not significantly affect the number of T cells and RBCs (Yélamos et al, Blood, The EMBO journal 25, 18, 2006; Farrés et al, Cell Death & Differentiation 22, 7, 2015). Thus, PARP1 inhibition is the main cause of potency and PARP2 inhibition is the main cause of toxicity. The development of highly selective PARP1 inhibitors may significantly reduce the toxicity produced by PARP2 without significantly reducing potency.

In summary, there is an urgent need to develop PARP inhibitors with high efficacy and good safety, especially inhibitors with high selectivity for PARP1.

### SUMMARY OF THE INVENTION

One of the purposes of the present invention is to provide a class of fused tricyclic compounds, which can be used as PARP1 inhibitors.

The second purpose of the present invention is to provide a class of pharmaceutical compositions, which contain the above-mentioned compounds as active ingredients and optional pharmaceutically acceptable carriers.

The third purpose of the present invention is to provide use of the above-mentioned compounds or compositions containing them in the preparation of PARP 1 inhibitors.

The fourth purpose of the present invention is to provide use of the above-mentioned compounds or compositions containing them in the preparation of drugs for preventing and/or treating diseases improved by inhibiting PARP1.

On the one hand, the present invention provides a compound of formula (I), a stereoisomer, a geometric isomer, a tautomer, a pharmaceutically acceptable salt or a hydrate thereof, 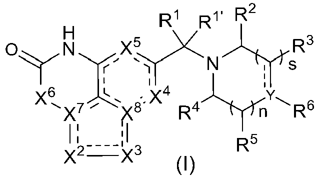 wherein,
-̅ -̅ -̅ -̅ -̅ is a single or double bond;
X² is selected from N, -C(=O) and CR^{8a};
X³ is selected from N, O and CR^{8b};
X⁴ is selected from N and CR⁹;
X⁵ is selected from N and CR¹⁰;
X⁶ is selected from NR^{7c} and CR¹⁴R¹⁵;
X⁷ and X⁸ are each selected from C and N, provided that X⁷ and X⁸ are not C at the same time; X² and X³ are not N at the same time;
R^{7c} is selected from hydrogen, substituted or unsubstituted C₁-C₆ alkyl, substituted or unsubstituted C₃-C₈ cycloalkyl, substituted or unsubstituted C₁-C₆ alkoxy, substituted or unsubstituted 3 to 8-membered heterocyclyl, substituted or unsubstituted C₂-C₆ alkynyl; preferably, R^{7c} is selected from hydrogen, C₁-C₄ alkyl being unsubstituted or substituted with halogen, hydroxyl or C₃-C₆ cycloalkyl, unsubstituted or halogen substituted C₃-C₆ cycloalkyl, C₁-C₆ alkoxy, 3 to 6 membered heterocyclyl, C₂-C₆ alkynyl;
R^{8a} and R^{8b} are each independently selected from hydrogen, halogen, cyano, substituted or unsubstituted C₁-C₆ alkyl, substituted or unsubstituted C₃-C₆ cycloalkyl; preferably, R^{8a} and R^{8b} are each independently selected from hydrogen, fluorine, chlorine, methyl, ethyl, cyclopropyl;
R⁹ and R¹⁰ are each independently selected from hydrogen, halogen, cyano, substituted or unsubstituted C₁-C₆ alkyl, substituted or unsubstituted C₁-C₆ alkoxy; preferably, R⁹ and R¹⁰ are each independently selected from hydrogen, halogen, cyano and C₁-C₄ alkyl; more preferably, R⁹ and R¹⁰ are each independently selected from hydrogen, fluorine, chlorine and methyl;
R¹⁴ and R¹⁵ are each independently selected from hydrogen, substituted or unsubstituted C₁-C₆ alkyl, substituted or unsubstituted C₃-C₆ cycloalkyl; or R¹⁴ and R¹⁵ together with the carbon atoms bonded thereto form C₃-C₆ cycloalkyl;
R¹, R^{1'}, R² and R³ are each independently selected from hydrogen, substituted or unsubstituted C₁-C₆ alkyl;
R⁴ and R⁵ are each independently selected from hydrogen, substituted or unsubstituted C₁-C₆ alkyl; or R⁴ and R⁵ together with the carbon atoms bonded thereto form C₃-C₆ cycloalkyl;
s, n are each independently selected from 0, 1 and 2;
Y is N, C or CH;
R⁶ is selected from: 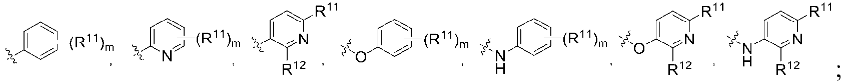
R¹¹ is independently selected from halogen, cyano, C₁-C₃ alkoxy, carbonyl, -CONHR¹³ and amino; preferably selected from halogen, -CONHR¹³ and cyano;
m is 0, 1, 2 or 3;
R¹² is selected from hydrogen, cyano, halogen, substituted or unsubstituted C₁-C₄ alkyl;
R¹³ is selected from hydrogen, substituted or unsubstituted C₁-C₆ alkyl, substituted or unsubstituted C₃-C₈ cycloalkyl, substituted or unsubstituted C₁-C₆ alkoxy, substituted or unsubstituted 3-8 membered heterocyclyl; preferably, R¹³ is selected from hydrogen, unsubstituted or halogen substituted C₁-C₄ alkyl, C₃-C₆ cycloalkyl, C₁-C₆ alkoxy;
wherein, the heterocyclyl refers to heterocyclic group containing 1-3 heteroatoms selected from N, O, and S; the substituted refers to being substituted by one or more selected from C₁-C₄ alkyl, C₁-C₆ alkoxy, halogen, hydroxyl, cyano, amino, carboxyl and C₃-C₆ cycloalkyl.

In some embodiments, X² is N or CR^{8a}, and the definitions of other substituents are the same as described above.

In some embodiments, X³ is N or CR^{8b}, and the definitions of other substituents are the same as described above.

In some embodiments, X⁷ is N, and the definitions of other substituents are the same as described above.

In some embodiments, X⁸ is N, and the definitions of other substituents are the same as described above.

In some embodiments, X⁴ is CH, and the definitions of other substituents are the same as described above.

In some embodiments, X⁵ is CR¹⁰, R¹⁰ is H, C₁-C₄ alkyl or halogen, and the definitions of other substituents are the same as described above.

In some embodiments, X⁶ is NR^{7c}, the definitions of other substituents are the same as described above, and the compound is not 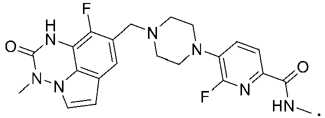 Preferably, R^{7c} is selected from hydrogen, C₁-C₄ alkyl being unsubstituted or substituted with halogen, hydroxyl or C₃-C₆ cycloalkyl, unsubstituted or halogen substituted C₃-C₆ cycloalkyl, C₁-C₆ alkoxy, 3 to 6 membered heterocyclyl, C₂-C₆ alkynyl.

In some embodiments, 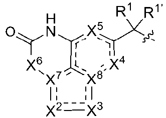 in formula (I) is selected from the following structures: 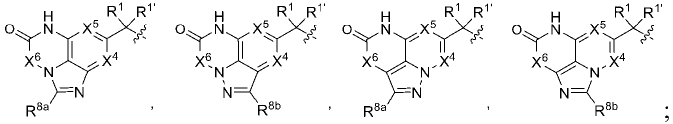 more preferably, selected from the following structures: 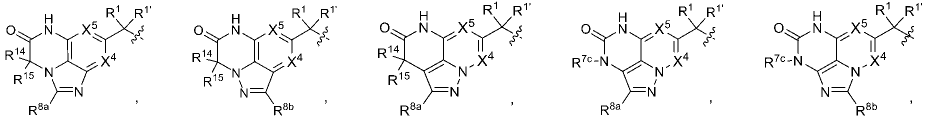 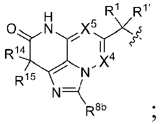 wherein the definitions of each substituent are the same as described above; further preferably, R^{8a} and R^{8b} are each independently selected from hydrogen, fluorine, chlorine, methyl, ethyl, cyano, and the definitions of other substituents are the same as described above.

In some embodiments, 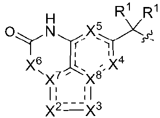 in formula (I) is selected from the following structures: 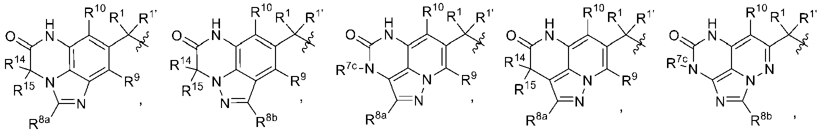 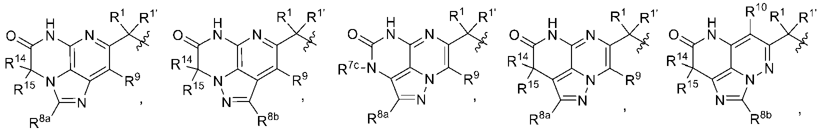 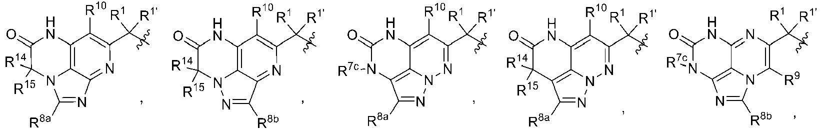 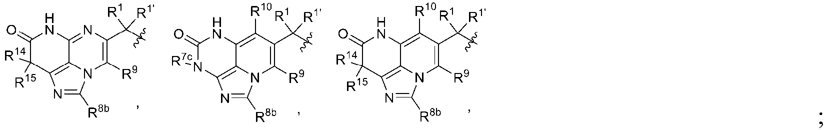 wherein, R^{8a} and R^{8b} are each independently selected from hydrogen, fluorine, chlorine, methyl, ethyl, cyano; R⁹ and R¹⁰ are each independently selected from hydrogen, fluorine, chlorine, methyl; and the definitions of other substituents are the same as described above. Preferably, R^{7c} is selected from hydrogen, C₁-C₄ alkyl, C₃-C₆ cycloalkyl, C₁-C₄ alkoxy.

In some embodiments, 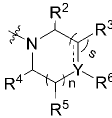 in formula (I) is selected from the following structures: 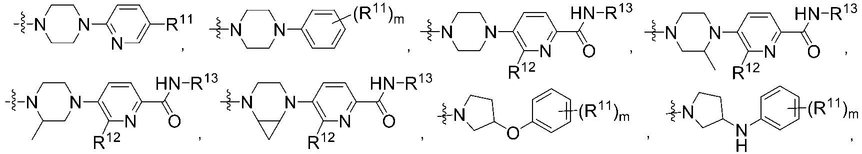 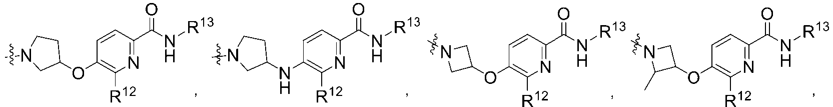 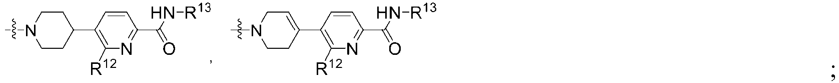 preferably, selected from the following structures: 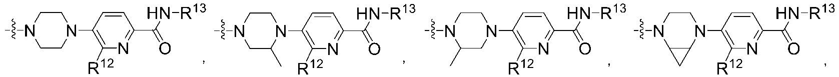 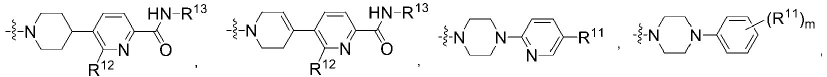 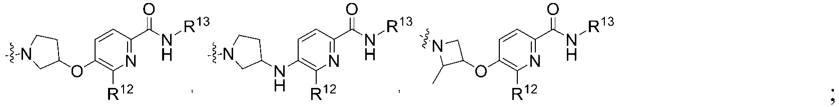 wherein, the definitions of the substituents of m, R¹¹, R¹², and R¹³ are the same as those in the formula (I).

In some embodiments, 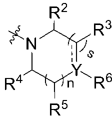 in formula (I) is selected from the following structures: 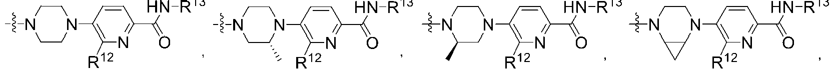 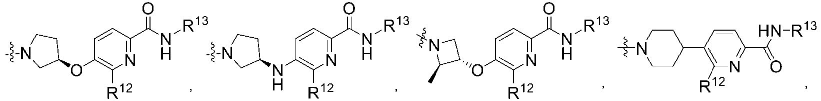 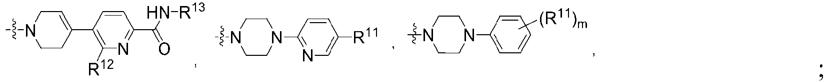 wherein, the definitions of each substituent are the same as above.

Those skilled in the art will understand that 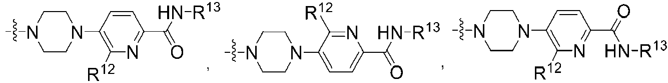 are equivalent.

In some embodiments, compounds of formula (II), formula (III), their stereoisomers, geometric isomers, tautomers, pharmaceutically acceptable salts or hydrates thereof are provided, 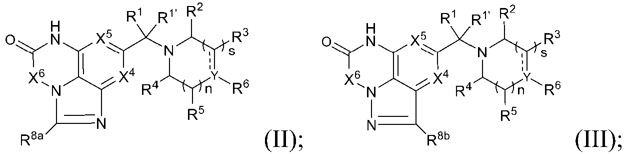 wherein the definitions of each substituent are the same as that of the formula (I).

In some embodiments, compounds of formula (IV), formula (V), their stereoisomers, geometric isomers, tautomers, pharmaceutically acceptable salts or hydrates thereof are provided, 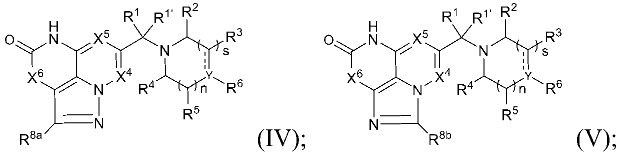 wherein, the definitions of each substituent are the same as that of the formula (I).

In some embodiments, in the compound of formula (I), formula (II), formula (III), formula (IV) or formula (V), X⁶ is NR^{7c}, and the definitions of each other substituent are the same as that of the above-mentioned formulas.

In some embodiments, in the compound of formula (I), formula (II), formula (III), formula (IV) or formula (V), X⁶ is CR¹⁴R¹⁵, and the definitions of each other substituent are the same as that of the above-mentioned formulas.

In another preferred embodiment, in the compound of formula (II) or formula (IV), R^{8a} is hydrogen or C₁-C₂ alkyl; preferably, R^{8a} is hydrogen or methyl.

In another preferred embodiment, in the compound of formula (III) or formula (V), R^{8b} is hydrogen or C₁-C₂ alkyl; preferably, R^{8b} is hydrogen or methyl.

Typical compounds of the present invention include but are not limited to:

| Comp ound No. | Structure | Comp ound No. | Structure |
|---|---|---|---|
| 5 | 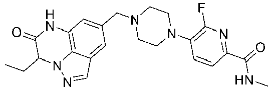 | 5-1 | 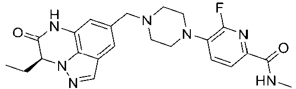 |
| 5-2 | 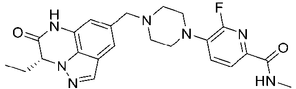 | 6 | 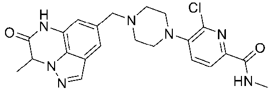 |
| 6-1 | 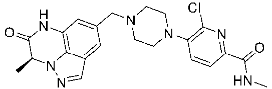 | 6-2 | 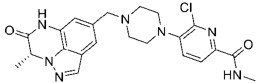 |
| 7 | 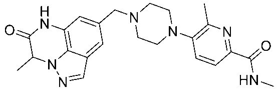 | 8 | 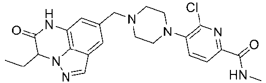 |
| 7-1 | 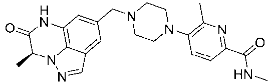 | 7-2 | 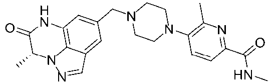 |
| 9 | 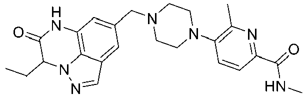 | 11 | 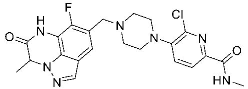 |
| 11-1 | 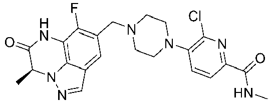 | 11-2 | 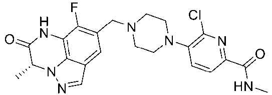 |
| 12 | 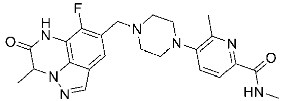 | 12-1 | 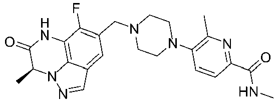 |
| 12-2 | 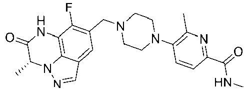 | 16 | 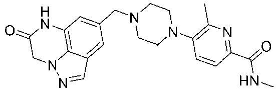 |
| 17 | 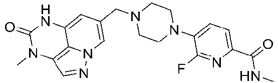 | 18 | 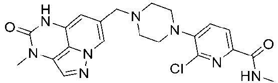 |
| 19 | 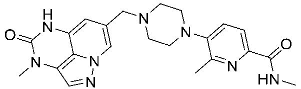 | 20 | 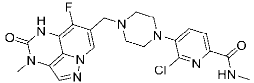 |
| 22 | 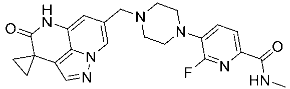 | 26 | 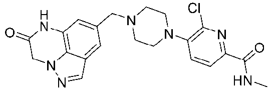 |
| 27 | 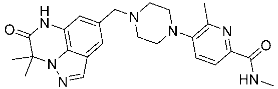 | 28 | 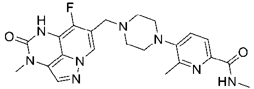 |
| 29 | 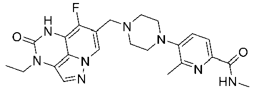 | 30 | 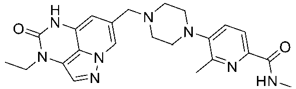 |
| 31 | 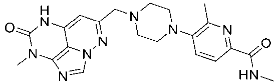 | 32 | 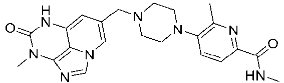 |
| 33 | 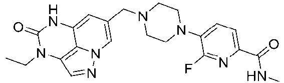 | 34 | 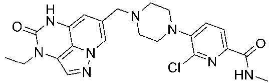 |
| 35 | 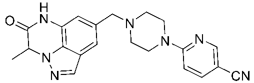 | 36 | 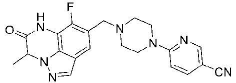 |
| 37 | 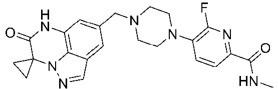 | 38 | 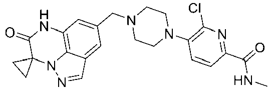 |
| 39 | 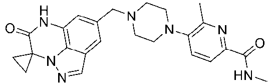 | 40 | 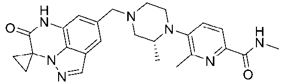 |
| 41 | 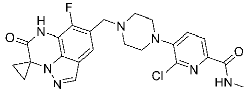 | 42 | 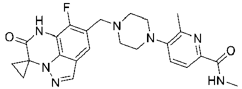 |
| 43 | 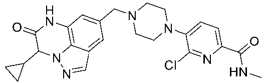 | 45 | 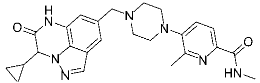 |
| 43-1 | 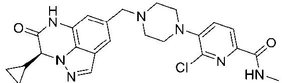 | 43-2 | 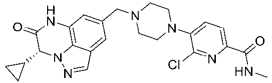 |
| 45-1 | 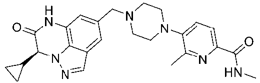 | 45-2 | 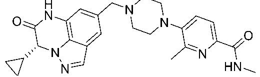 |
| 47 | 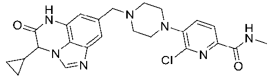 | 49 | 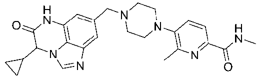 |
| 47-1 | 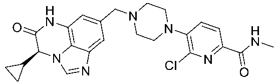 | 47-2 | 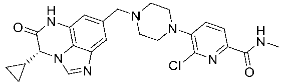 |
| 49-1 | 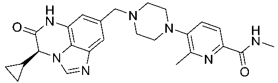 | 49-2 | 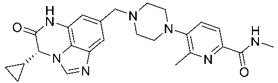 |
| 51 | 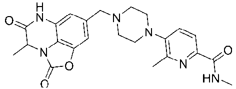 | 52 | 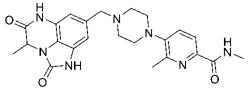 |
| 53 | 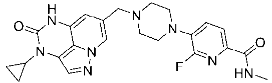 | 54 | 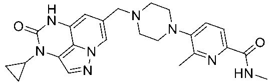 |
| 55 | 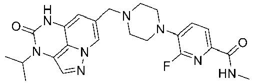 | 56 | 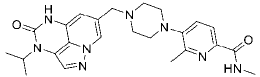 |
| 57 | 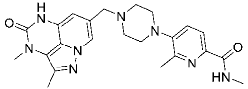 | 58 | 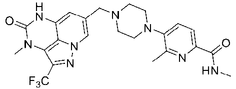 |
| 59 | 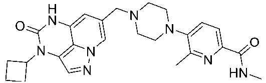 | 60 | 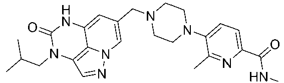 |
| 61 | 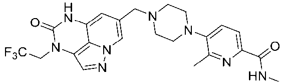 | 62 | 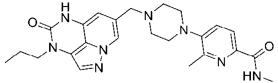 |
| 63 | 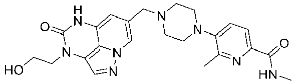 | 64 | 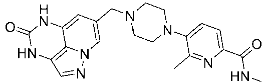 |
| 65 | 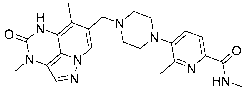 | 66 | 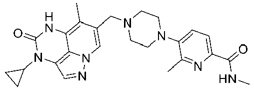 |
| 67 | 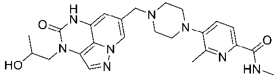 | 68 | 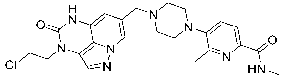 |
| 69 | 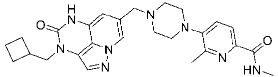 | 70 | 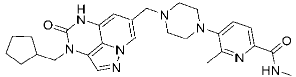 |
| 71 | 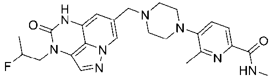 | 72 | 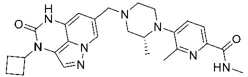 |
| 73 | 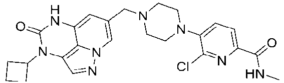 | 74 | 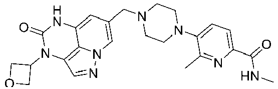 |
| 75 | 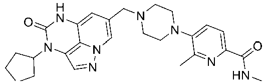 | 76 | 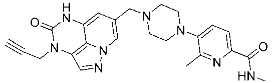 |
| 77 | 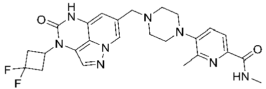 | 78 | 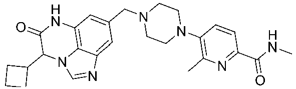 |
| 79 | 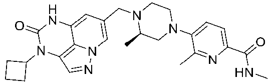 | 80 | 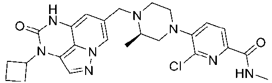 |
| 81 | 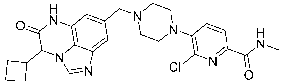 | 82 | 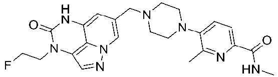 |

The compounds of formula (I), (II), (III), (IV), (V), each specific compounds, their stereoisomers, geometric isomers, tautomers, pharmaceutically acceptable salts, and hydrates thereof described in the present invention are collectively referred to as the compounds of the present invention.

The present invention also provides a method for preparing the compounds of the present invention, intermediates for preparing the compounds of the present invention, and a method for preparing the intermediates.

The present invention also provides a composition, comprising at least one of the compounds of the present invention.

Another aspect of the present invention provides a pharmaceutical composition comprising a therapeutically effective amount of one or more selected from the compounds of the present invention, and an optional pharmaceutically acceptable carrier.

In one embodiment, the present invention provides a pharmaceutical composition comprising a therapeutically effective amount of the compounds of the present invention or the pharmaceutically acceptable salts thereof.

In another embodiment, the present invention provides a combination, particularly a pharmaceutical combination, comprising a therapeutically effective amount of the compounds of the present invention, and one or more other therapeutic agents.

In certain embodiments of the pharmaceutical composition, the pharmaceutical composition is formulated for intravenous administration, intramuscular administration, oral administration, rectal administration, inhalation administration, nasal administration, topical administration, ocular administration, or aural administration. In other embodiments of the pharmaceutical composition, the pharmaceutical composition is a tablet, a pill, a capsule, a liquid, an inhaler, a nasal spray solution, a suppository, a solution, an emulsion, an ointment, an eye drops or an ear drop. In other embodiments of the pharmaceutical composition, it also contains one or more additional therapeutic agents.

The pharmaceutical composition of the present invention can be selected to contain at least one selected from the following pharmaceutically acceptable carriers according to the mode of administration and the nature of the dosage form: diluent, adjuvant, excipient, preservative, filler, binder, disintegrant, wetting agent, emulsifier, suspending agent, sweetener, flavoring agent, fragrance, antibacterial agent, antifungal agent, lubricant, dispersant, thermosensitive material, temperature regulator, adhesive, stabilizer, suspending agent, etc.

The compounds of the present invention can be used alone, in combination with other compounds of the present invention, or in combination with one or more, preferably one or two, other substances simultaneously or sequentially.

Another aspect of the present invention provides the use of the compounds of the present invention or the above-mentioned pharmaceutical composition in the preparation of PARP1 inhibitors.

In another aspect, the present invention provides the use of the compounds of the present invention or the above-mentioned pharmaceutical composition in the preparation of drugs for preventing and/or treating a disease improved by inhibiting PARP1.

In another aspect, the present invention provides a method for preventing and/or treating a disease improved by inhibiting PARP1, the method comprising administering an effective amount of one or more selected from the compounds of the present invention or the pharmaceutical composition to an individual in need of such treatment.

In some embodiments of the present invention, the disease improved by inhibiting PARP1 includes but is not limited to cancer.

In some embodiments of the present invention, the cancer includes but is not limited to malignant tumors, such as any of ovarian cancer, breast cancer, fallopian tube cancer, endometrial cancer, peritoneal cancer, gastric cancer, colon cancer, bladder cancer, pancreatic cancer, biliary cancer, osteosarcoma, cervical cancer, head and neck tumors, germ cell and embryonic cancer, esophageal cancer, malignant glioma, Ewing sarcoma, pancreatic cancer, melanoma, bile duct cancer, prostate cancer, small cell lung cancer, non-small cell lung cancer, lymphoma and blood cancer.

In some embodiments of the present invention, the genome of the cancer is a type lacking homologous recombination repair.

In some embodiments of the present invention, the cancer is dependent on a pathway where double-strand DNA damage is repaired without homologous recombination.

In some embodiments of the present invention, the cancer comprises one or more cancer cells that lack the ability to repair double-strand DNA breaks through homologous recombination relative to normal cells.

In some embodiments of the present invention, the cancer comprises one or more cancer cells that lack BRCA1 or BRCA2, or that have BRCA1 or BRCA2 mutation.

### Terminology

In the present invention, unless otherwise explicitly stated, the terms used in the present invention have the meanings defined below. Terms not explicitly defined in the present invention have the general meanings generally understood by those skilled in the art.

When a group is accompanied by a wavy line " 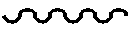 ", the wavy line indicates the position of connection between the group and the rest of the molecule.

As used herein, "- - - -" represents a single bond or a double bond. It is within the ability of those skilled in the art to determine whether - - - - represents a single bond or a double bond based on the valence of the relevant ring atoms and the groups bonded, etc. It is understood by those skilled in the art that the relevant rings may be saturated, partially saturated or aromatic.

The terms "option", "optional" or "optionally" used herein mean that the substitution pattern, event or situation described subsequently may or may not occur, and the description includes situations where the substitution pattern occurs and situations where the substitution pattern does not occur.

As used herein, "halogen" may be fluorine, chlorine, bromine or iodine. Preferred halogens are fluorine or chlorine.

As used herein, "C₁-C₆ alkyl" refers to a fully saturated straight or branched hydrocarbon group having 1-6 carbon atoms; specific examples thereof may include methyl, ethyl, propyl, n-propyl, isopropyl, butyl, n-butyl, isobutyl, tert-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethyl-butyl, cyclopropylmethyl, cyclobutylmethyl, cyclopentylmethyl, and the like, but are not limited thereto. The meaning of C₁-C₄ alkyl is analogous.

As used herein, " C₂-C₆ alkynyl" refers to a straight or branched monovalent hydrocarbon group having 2-6 carbon atoms and containing at least one triple bond. Representative examples include, but are not limited to, ethynyl, propynyl, iso-propynyl, butynyl, iso-butynyl, pentynyl, iso-pentynyl, hexynyl, and the like.

As used herein, " C₁-C₆ alkoxy" refers to a RO-group, wherein R is a C₁-C₆ alkyl group as described above. Specific examples of alkoxy include methoxy, ethoxy, n-propoxy, iso-propoxy, and the like. The meaning of C₁-C₃ alkoxy is analogous to that of the above.

As used herein, "C₃-C₈ cycloalkyl" refers to a fully saturated monovalent cyclic hydrocarbon compound group containing 3-8 ring carbon atoms, and specific examples thereof include cyclopropyl, cyclobutyl, cyclopentyl, cyclohexyl, and the like. The meaning of C₃-C₆ cycloalkyl is analogous to that of the above.

As used herein, "3-8 membered heterocyclyl" refers to a 3-8 membered non-aromatic cyclic group containing 1 to 3 heteroatoms selected from nitrogen, oxygen, and sulfur on the ring, and specific examples thereof include oxetane, tetrahydroimidazole, and tetrahydrofuran.

As used herein, "pharmaceutically acceptable salt" refers to a salt that retains the biological effects and properties of a compound, and the salt is not biologically or otherwise undesirable. Non-limiting examples of the salt include non-toxic, inorganic or organic, base or acid addition salts of the compounds of the present invention. In many cases, due to the presence of amino and/or carboxyl groups or groups similar thereto, the compounds of the present invention are able to form acid salts and/or base salts. Pharmaceutically acceptable acid addition salts can be formed with inorganic or organic acids, inorganic acids including, for example, hydrochloric acid, hydrobromic acid, sulfuric acid, nitric acid, phosphoric acid, etc.; organic acids including, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, etc. Pharmaceutically acceptable base addition salts may also be formed with inorganic or organic bases, including, for example, sodium, potassium, lithium, ammonium, calcium, magnesium, iron, zinc, copper, manganese, aluminum, and the like; ammonium, potassium, sodium, calcium, and magnesium salts are particularly preferred; organic bases include, for example, primary, secondary, and tertiary amines, substituted amines (including naturally occurring substituted amines), cyclic amines, basic ion exchange resins, and the like, particularly, for example, isopropylamine, trimethylamine, diethylamine, triethylamine, tripropylamine, and ethanolamine. Pharmaceutically acceptable salts of the present invention may be synthesized from the parent compound (basic or acidic moiety) by conventional chemical methods.

The compounds of the present invention may contain one or more asymmetric centers and may therefore give rise to enantiomers, diastereomers, and other stereoisomeric forms, which may be defined as (R)- or (S)-, or as (D)- or (L)- for amino acids, according to absolute stereochemistry. It is intended herein to include all such possible isomers and their racemic and optically pure forms, whether or not they are specifically described herein.

"Stereoisomer" refers to a compound composed of the same atoms bonded by the same bonds but having different three-dimensional structures that are not interchangeable. Various stereoisomers and mixtures thereof are contemplated herein, and include "enantiomers", which refer to two stereoisomers whose molecules are non-superimposable mirror images of each other.

"Tautomer" refers to a proton transfer from one atom of a molecule to another atom of the same molecule. The present invention includes tautomers of any of the compounds described.

The compounds of the present invention or their stereoisomers, geometric isomers, tautomers, and pharmaceutically acceptable salts thereof may also exist in the form of polymorphs, solvates (e.g., hydrates), and prodrugs, which are also included within the scope of the present invention.

As used herein, the term "solvate" means a stoichiometric or non-stoichiometric solvent addition form. If the solvent is water, the solvate formed is a hydrate, and when the solvent is ethanol, the solvate formed is an ethanolate. Hydrates are formed by one or more molecules of water and one molecule of the substance, wherein the water retains its molecular state of H₂O, and such a combination can form one or more hydrates, such as hemihydrates, monohydrates and dihydrates.

As used herein, "prodrugs" refer to chemically modified active or inactive compounds, which, after being administered to an individual, undergo physiological actions in the body (such as hydrolysis, neogenesis, etc.) to become compounds of the present invention. The adaptability and technology of making and using prodrugs are well known to those skilled in the art.

As used herein, the term "therapeutically effective amount" refers to the amount of the compound of the present invention that can cause a biological or medical response in an individual or improve symptoms, slow down or delay the progression of a disease, or prevent a disease, etc.

As used herein, the term "subject" refers to an animal. Preferably, the animal is a mammal. The subject also refers to, for example, primates (such as humans), cattle, sheep, goats, horses, dogs, cats, rabbits, rats, mice, fish, birds, etc. In a preferred embodiment, the subject is a human.

As used herein, the term "inhibit" refers to the alleviation or suppression of a particular condition, symptom or disorder or disease, or a significant reduction in the baseline activity of a biological activity or process.

As used herein, in one embodiment, the term "treating" any disease or condition refers to ameliorating the disease or condition (i.e., preventing or slowing the development of the disease or at least one clinical symptom thereof). In another embodiment, "treating" refers to improving at least one physical parameter, which may not be perceived by the patient. In another embodiment, "treating" refers to regulating a disease or condition physically (e.g., stabilizing a perceptible symptom) or physiologically (e.g., stabilizing a physical parameter), or both.

### Beneficial Effects

The compounds of the present invention have high selectivity for PARP1. In specific embodiments, the compounds of the present invention have a selectivity fold of ≥100, preferably ≥200, more preferably ≥500, even more preferably ≥800, and most preferably ≥1000 for PARP1/2; and, compared with olaparib (AZD-2281), the side effects are fewer, and the clinical application value is high.

The present invention has been described in detail above, but the above embodiments are only illustrative in nature and are not intended to limit the present invention. In addition, this article is not limited by any theory described in the prior art or invention content or the following examples.

### DETAILED DESCRIPTION OF THE INVENTION

The present invention is further described below in conjunction with the examples. It should be noted that the following examples are provided for illustrative purposes only and do not constitute a limitation on the scope of protection claimed in the present invention.

The experimental materials and reagents used in the following examples can be obtained from commercial channels unless otherwise specified. The raw materials can usually be obtained from commercial sources or easily prepared by methods known to those skilled in the art.

In each example, the experimental instruments or materials used are as follows:
¹H NMR was recorded by a Bruker Advance -400 or -600 nuclear magnetic resonance instrument, and the chemical shift was expressed in δ (ppm); the mass spectrum was recorded by a Finnigan/MAT-95 (EI) and Finnigan LCQ/DECA and Micromass Ultra Q-TOF (ESI) mass spectrometer; the silica gel used for reverse phase preparative HPLC separation was 200-300 mesh.

### Abbreviations:

PE: petroleum ether; EA: ethyl acetate; DIEA: N,N-diisopropylethylamine; DCM: dichloromethane; DMF: N,N-dimethylformamide; XPhos Pd G2: chloro(2-dicyclohexylphosphino-2',4',6'-triisopropyl-1,1'-biphenyl)[2-(2'-amino-1,1'-biphenyl)]palladium(II); Ruphos Pd G3: methanesulfonic acid(2-dicyclohexylphosphino-2',6'-diisopropyloxy-1,1'-biphenyl)(2-amino-1,1'-biphenyl-2-yl)palladium(II).

### Example

### Synthesis of key intermediates

### Intermediate 1a: synthesis of 6-chloro-N-methyl-5-(piperazine-1-yl)picolinamide hydrochloride

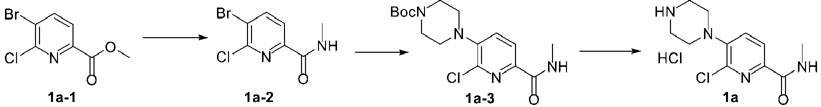

### Step 1: synthesis of 5-bromo-6-chloro-N-methylpicolinamide

**1a-1** (1.00 g, 3.99 mmol) and ethanol solution of methylamine (33 wt%, 10 mL) were added to a flask in sequence. The reaction was carried out at rt for 16 h. The reaction solution was concentrated to obtain **1a-2** (0.94 g, yellow oil), yield: 94.37%, LCMS (ESI): m/z 250.9 [M+H]⁺; RT=1.448 min (2.50 min) (peak time before brackets, total running time in brackets).

### Step 2: synthesis of tert-butyl 4-(2-chloro-6-(methylcarbamoyl)pyridin-3-yl)piperazine-1-carboxylate

**la-2** (620 mg, 2.49 mmol), toluene (15 mL), tert-butyl piperazine-1-carboxylate (370 mg, 1.99 mmol), 1,1'-binaphthyl-2,2'-bisdiphenylphosphine (155 mg, 0.25 mmol), cesium carbonate (2020 mg, 6.21 mmol) and palladium acetate (56 mg, 0.25 mmol) were added to a flask in sequence. The reaction was carried out at 100°C under nitrogen for 16 h. The reaction solution was filtered, and the filtrate was concentrated. Purification by silica gel column chromatography (PE: EA= 1:1) to obtain **1a-3** (180 mg, yellow oil), yield: 20.41%, LCMS (ESI): m/z 355.1 [M+H]⁺; RT=1.679 min (2.50 min). ¹H-NMR (400 MHz, DMSO-d₆): δ 8.46 (d, J=4.8 Hz, 1H), 7.95 (d, J=8.4 Hz, 1H), 7.68 (d, J=8.4 Hz, 1H), 3.50 (s, 4H), 3.06-3.04 (m, 4H), 2.80 (d, J=4.8 Hz, 3H), 1.43 (s, 9H).

### Step 3: synthesis of 6-chloro-N-methyl-5-(piperazin-1-yl)picolinamide hydrochloride

**1a-3** (180 mg, 0.51 mmol), DCM (2 mL) and dioxane hydrochloride (4.0 M, 2 mL) were added to a dry flask. After stirring at rt for 2 h, the mixture was concentrated to obtain **1a** (140 mg, yellow solid). Yield: 94.59%, LCMS (ESI): m/z 255.1 [M+H]⁺; RT=0.336 min & 0.461 min (2.50 min). ¹H-NMR (400 MHz, DMSO-d₆): δ 9.40 (s, 2H), 8.50 (d, J=4.4 Hz, 1H), 7.97 (d, J=8.4 Hz, 1H), 7.76 (d, J=8.4 Hz, 1H), 3.34-3.32 (m, 4H), 3.25 (m, 4H), 2.80 (d, J=4.8 Hz, 3H).

### Intermediate 2a: synthesis of N-methyl-5-(piperazin-1-yl)pyridineamide hydrochloride

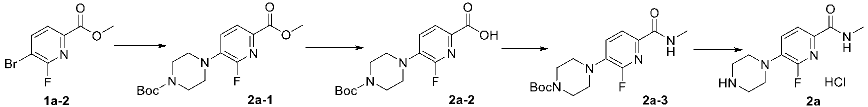

### Step 1: synthesis of tert-butyl 4-(2-fluoro-6-(methoxycarbonyl)pyridin-3-yl)piperazine-1-carboxylate

**la-2** (500 mg, 2.1 mmol), 1-tert-butyloxycarbonylpiperazine (600 mg, 3.2 mmol), Ruphos Pd G3 (180 mg, 0.21 mmol), cesium carbonate (1.7 g, 5.2 mmol), and dioxane (15 mL) were added to a 50 mL flask and the mixture was heated at 80°C overnight under nitrogen. The reaction solution was concentrated and the residue was purified by silica gel column (PE:EA=2:1) to obtain **2a-1** (640 mg, yellow solid) with a yield of 88%. LCMS (ESI): m/z 340.1[M+H]⁺; RT= 1.74 min (3.00 min).

### Step 2: synthesis of 5-(4-(tert-butyloxycarbonyl)piperazine-1-yl)-6-fluoropicolinic acid

To a mixture of **2a-1** (320 mg, 0.94 mmol) in THF (8 mL) was added lithium hydroxide monohydrate (200 mg, 4.7 mmol) aqueous solution (8 mL) dropwise. The mixture was stirred for 2 h. Adjust the pH of the reaction solution to 6 with 1M hydrochloric acid solution, concentrate the solution and purify it with a reverse phase column (2%~40% acetonitrile/H₂O) to obtain **2a-2** (300 mg, yellow solid), yield: 92%. LCMS (ESI): m/z 326.1[M+H]⁺; RT= 1.26 min (3.00 min).

### Step 3: synthesis of tert-butyl 4-(2-fluoro-6-(methylcarbamoyl)pyridin-3-yl)piperazine-1-carboxylate

**2a-2** (300 mg, 0.92 mmol), 1-hydroxybenzotriazole (149 mg, 1.1 mmol), 1-(3-dimethylaminopropyl)-3-ethylcarbodiimide hydrochloride (211 mg, 1.1 mmol) and DIEA (237 mg, 1.84 mmol) in DMF (6 mL) was added methylamine hydrochloride (123 mg, 1.84 mmol) and the mixture was stirred overnight. The reaction solution was added water (20 mL), extracted with EA (30 mL) three times. The organic phase was washed with saturated brine (20 mL), dried over anhydrous sodium sulfate, filtered. The filtrate was concentrated. The residue was purified by reverse phase column (20%~70% acetonitrile/H₂O) to obtain **2a-3** (250 mg, white solid), yield: 80%. LCMS (ESI): m/z 283.1 [M-100+H]⁺; RT= 1.66 min (3.00 min).

### Step 4: synthesis of 6-fluoro-N-methyl-5-(piperazine-1-yl)pyridineamide hydrochloride

In a dry 20 mL single-necked flask at rt, 2a-3 (250 mg, 0.74 mmol), EA (3 mL), 4M hydrochloric acid dioxane solution (1 mL) were added in sequence. The mixture was stirred at rt for 2 h, and concentrated to obtain product **2a** (200 mg, yellow solid), yield: 100%. LCMS (ESI): m/z 239.1 [M+H]⁺; RT= 0.91 min (3.00 min). ¹H-NMR (600 MHz, CD₃OD): 7.98-7.96 (m, 1H), 7.67-7.64 (m, 1H), 3.50-3.48 (m, 4H), 3.45-3.43 (m, 4H), 2.93 (d, J=3.6 Hz, 3H).

### Intermediate 3a: synthesis of N,6-dimethyl-5-(piperazine-1-yl)picolinamide hydrochloride

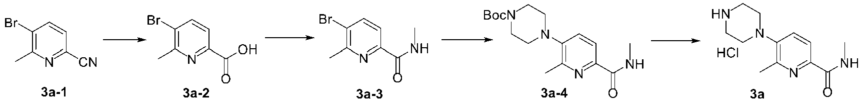

### Step 1: synthesis of 5-bromo-6-methylpicolinic acid

The mixture of **3a-1** (500 mg, 2.54 mmol), and sodium hydroxide (507 mg, 12.69 mmol) in methanol (6 mL)/H₂O (3 mL) was stirred at 70°C for 1 h. The reaction solution was concentrated. Dilute with water (10 mL), adjust to pH=4 with 3 M dilute hydrochloric acid, then filter. Collect the filter cake to obtain **3a-2** (300 mg, white solid), yield: 54.72%, LCMS (ESI): m/z 218.0 [M+H]⁺; RT=1.208 min (2.50 min). ¹H-NMR (400 MHz, DMSO-d₆): δ 8.12 (d, J=8.0 Hz, 1H), 7.78 (d, J=8.4 Hz, 1H), 2.67 (s, 3H).

### Step 2: synthesis of 5-bromo-N,6-dimethylpicolinamide

**3a-2** (300 mg, 1.39 mmol) and O-(7-azabenzotriazole-1-yl)-N,N,N',N'-tetramethyluronium hexafluorophosphate (792 mg, 2.08 mmol) in DMF (3 mL) was added DIEA (0.92 mL, 5.55 mmol) and methylamine tetrahydrofuran solution (2.0 M, 1.39 mL, 2.78 mmol). The reaction was carried out at rt for 1 h. Dilute with water (30 mL) and extract with EA (10 mL×2). The organic phase was washed with saturated brine, dried over anhydrous sodium sulfate, filtered. The filtrate was concentrated under reduced pressure. The residue was purified by prep-silica gel plate (PE:EA=3:2) to obtain **3a-3** (215 mg, yellow solid), yield: 67.59%, LCMS (ESI): m/z 231.0 [M+H]⁺; RT=1.427 min (2.50 min). ¹H-NMR (400 MHz, DMSO-d₆): δ 8.67 (d, J=4.0 Hz, 1H), 8.18 (d, J=8.4 Hz, 1H), 7.75 (d, J=8.4 Hz, 1H), 2.82 (d, J=4.8 Hz, 3H), 2.65 (s, 3H).

### Step 3: synthesis of tert-butyl 4-(2-methyl-6-(methylcarbamoyl)pyridin-3-yl)piperazine-1-carboxylate

The mixture **of3a-3** (200 mg) was added to a dry flask. mg, 0.87 mmol), tert-butyl piperazine-1-carboxylate (179 mg, 0.96 mmol), 1,1'-binaphthyl-2,2'-bisdiphenylphosphine (54 mg, 0.09 mmol), cesium carbonate (711 mg, 2.18 mmol) and palladium acetate (20 mg, 0.09 mmol) in toluene (8 mL) was stirred at 100°C for 16 h under nitrogen. The reaction solution was filtered, and the filtrate was concentrated. The residue was purified by preparative plate (PE:EA=1:1) to obtain **3a-4** (160 mg, yellow solid), yield: 54.80%, LCMS (ESI): m/z 335.1 [M+H]⁺.

### Step 4: synthesis of N,6-dimethyl-5-(piperazin-1-yl)picolinamide hydrochloride

**3a-4** (160 mg, 0.48 mmol), DCM (2 mL) and HCl/dioxane (4.0 M, 2 mL) were added to a dry flask. After 4 h of reaction at rt, the product was concentrated to obtain **3a** (129 mg, yellow solid). Yield: 99.58%, LCMS (ESI): m/z 235.2 [M+H]⁺; RT=0.340 min & 0.450 min (2.50 min). ¹H-NMR (400 MHz, DMSO-d₆): δ 9.44 (s, 2H), 8.56 (d, J=4.8 Hz, 1H), 7.88 (d, J=8.0 Hz, 1H), 7.61 (d, J=8.4 Hz, 1H), 3.25-3.16 (m, 8H), 2.82 (d, J=4.4 Hz, 3H), 2.54 (s, 3H).

### Intermediate 4a: synthesis of N-ethyl-6-fluoro-5-(piperazine-1-yl)picolinamide hydrochloride

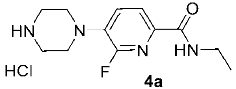

The synthesis method is similar to that of intermediate **2a,** except that ethylamine was used instead of methylamine hydrochloride. LCMS(ESI): m/z 253.2 [M+H]⁺; RT=0.527 min (2.5 min).

### Intermediate 5a: synthesis of N-cyclopropyl-6-fluoro-5-(piperazine-1-yl)picolinamide hydrochloride

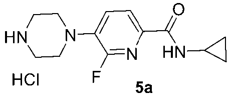

The synthesis method is similar to that of intermediate **2a,** except that cyclopropylamine was used instead of methylamine hydrochloride. LCMS(ESI): m/z 264.2 [M+H]⁺; RT=0.958 min (2.5 min).

### Intermediate 6a: 7-fluoro-8-(hydroxymethyl)-2,4-dimethyl-4H-imidazo[1,5,4-de]quinoxaline-5(6H)-one

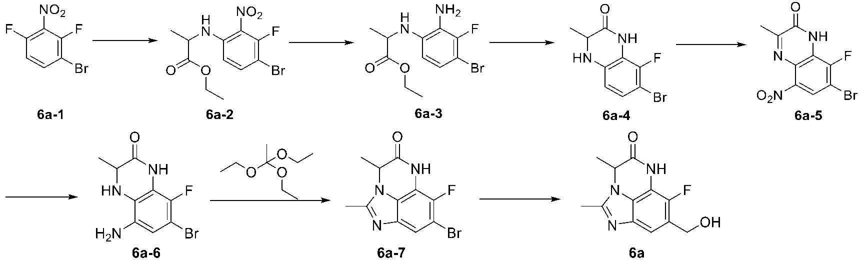

### Step 1: synthesis of (4-bromo-3-fluoro-2-nitrophenyl)alanine ethyl ester

**6a-1** (5 g, 21 mmol) in DMF (25 mL) was added DL-alanine ethyl ester hydrochloride (2.89 g, 18.9 mmol) and DIEA (8.1 g, 63 mmol) under ice bath. The mixture was warmed to rt and stirred overnight. Add water (100 mL) to the reaction solution, extract with EA (60 mL) three times. The organic phase was concentrated. The residue was purified by silica gel column (EA:PE=1:10) to obtain **6a-2** (2.6 g, 7.76 mmol), orange solid, yield: 37%. ¹H NMR (600 MHz, CDCl₃) δ 7.47 (dd, J=9.2, 7.0 Hz, 1H), 6.41 (dd, J=9.4, 1.6 Hz, 1H), 4.24 (q, J=7.0 Hz, 2H), 4.18 (t, J=7.0 Hz, 1H), 1.56 (d, J=7.0 Hz, 3H), 1.28 (t, J=7.0 Hz, 3H).

### Step 2: synthesis of (2-amino-4-bromo-3-fluorophenyl)alanine ethyl ester

**6a-2** (600 mg, 1.79 mmol) and ammonium chloride (949 mg, 17.9 mmol) were added to a solution of methanol (20 mL) and water (5 mL). Under ice bath, zinc powder (1.16 g, 17.9 mmol) was added in batches and stirred the mixture at rt for 2 h. The reaction solution was filtered, the solid was washed with methanol. The filtrate was concentrated and diluted with EA (50 mL), alkalized with saturated sodium bicarbonate aqueous solution. The aqueous phase was extracted three times with EA, and the organic phase was concentrated to obtain crude product **6a-3** (304 mg, 0.996 mmol) as a brown solid, yield: 55.6%. LCMS (ESI): m/z 307.0 [M+H]⁺; RT= 1.70 min (3.00 min).

### Step 3: synthesis of 7-bromo-8-fluoro-3-methyl-3,4-dihydroquinoxaline-2(1H)-one

**6a-3** in methanol (15 mL) and EA (10 mL) was added HCl/dioxane (4M, 2 mL). The mixture was stirred at rt for 2 h, concentrated to obtain crude product **6a-4** (260 mg, 0.996 mmol), LCMS (ESI): m/z 257.0 [M-H]⁻; RT = 1.38 min (3.00 min).

### Step 4: synthesis of 7-bromo-8-fluoro-3-methyl-5-nitroquinoxaline-2(1H)-one

**6a-4** (260 mg, 1.0 mmol) dissolved in concentrated sulfuric acid (8 mL, 95% aq.) was added potassium nitrate (121 mg, 1.2 mmol) in an ice-water bath. The mixture was stirred for 2 h, slowly added to ice water. An orange-red solid precipitated. The solid was collected by filtration, washed with water, and then dried to obtain **6a-5** (125 mg, 0.42 mmol). The crude product was used directly in the next step without purification. LCMS (ESI): m/z 299.9 [M-H]⁻; RT= 1.33 min (3.00 min).

### Step 5: synthesis of 5-amino-7-bromo-8-fluoro-3-methyl-3,4-dihydroquinoxaline-2(1H)-one

To the mixture of **6a-5** (251 mg, 0.831mmol) and ammonium chloride (265 mg, 5 mmol) in methanol (10mL)/water (1mL) was added zinc powder (325 mg, 5 mmol) under stirring. Stir the mixture at rt for 3 h, filter, and wash the solid with methanol. The filtrate was concentrated, diluted with EA (20mL), which was alkalized with saturated sodium bicarbonate aqueous solution. Extract the aqueous phase with EA three times. The combined organic phase was washed with saturated brine (20mL), dried with anhydrous sodium sulfate, filtered, and concentrated to obtain **6a-6** (130 mg) as a brown solid. LCMS (ESI): m/z 275.9 [M+H]⁺; RT= 2.49 min (5.00 min).

### Step 6: synthesis of 8-bromo-7-fluoro-2,4-dimethyl-4H-imidazo[1,5,4-de]quinoxaline-5(6H)-one

**6a-6** (130 mg) in triethyl orthoacetate (5 mL) was heated to 80°C and the mixture was stirred for 1 h under nitrogen. After cooled to rt, acetic acid (3 mL) was added, and the mixture was continued to heat to 100°C for 2 h. The reaction solution was concentrated to and the crude product was dissolved in DMSO (3 mL) and purified by reverse phase column (acetonitrile: water (1‰ ammonium bicarbonate = 0%-80%)) to obtain **6a-7** (30 mg, 0.101 mmol), white solid. LCMS (ESI): m/z 298.1 [M+H]⁺; RT= 1.25 min (3.00 min). ¹H NMR (600 MHz, DMSO-d₆): δ 11.35 (s, 1H), 7.42 (d, J=4.8 Hz, 1H), 5.34 (q, J=7.0 Hz, 1H), 2.54 (d, J=7.9 Hz, 3H), 1.64 (d, J=7.0 Hz, 3H).

### Step 7: synthesis of 7-Fluoro-8-(hydroxymethyl)-2,4-dimethyl-4H-imidazo[1,5,4-de]quinoxalin-5(6H)-one

**6a-7** (30 mg, 0.101 mmol), (tributyltin)methanol (65 mg, 0.202 mmol) and x-phos Pd G2 (16 mg, 0.0202 mmol) in dioxane (5 mL) was stirred at 110°C for 2 h under nitrogen. The reaction solution was concentrated and purified by silica gel column (methanol: DCM = 1: 10) to obtain product **6a** (15 mg, 0.0602 mmol) as a white solid, yield: 59.6%. LCMS (ESI): m/z 250.2 [M+H]⁺; RT = 0.93 min (3.00 min).

### Intermediate 9a: 8-(Hydroxymethyl)-3-methyl-1H-pyrazolo[1,5,4-de]quinoxaline-2(3H)-one

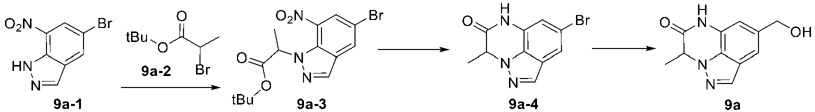

### Step 1: synthesis of tert-butyl 2-(5-bromo-7-nitro-1H-indazol-1-yl)propanoate

**9a-1** (1 g, 4.13 mmol) in DMF (20 mL) was added **9a-2** (950 mg, 4.54 mmol) and potassium carbonate (1.7 g, 12.4 mmol). The mixture was stirred for 2 h, diluted with EA (60 mL). The EA phase was washed with saturated brine, dried over anhydrous sodium sulfate, concentrated, and purified by silica gel column (EA:PE=1:5) to obtain **9a-3** (480 mg, 1.30 mmol), yellow oily solid, yield: 31%. ¹H NMR (400 MHz, DMSO-d₆): δ 8.55 (d, J=1.8 Hz, 1H), 8.45 (s, 1H), 8.31 (d, J=1.8 Hz, 1H), 5.57 (q, J=7.1 Hz, 1H), 1.83 (d, J=7.1 Hz, 3H), 1.31 (s, 9H).

### Step 2: synthesis of 8-bromo-3-methyl-1H-pyrazolo[1,5,4-de]quinoxalin-2(3H)-one

**9a-3** (1.05 g, 2.96 mmol) in acetic acid (10 mL) was added iron powder (994 mg, 17.7 mmol). The mixture was stirred at 80°C for 2 h, filtered through diatomaceous earth. The filtrate was concentrated, diluted with EA (30mL). EA phase was washed with saturated sodium bicarbonate aqueous solution, saturated brine, dried over anhydrous sodium sulfate, concentrated, and purified by silica gel column (EA:PE=1:3) to obtain product **9a-4** (604 mg, 1.86 mmol), white solid, yield: 63%.

### Step 3: synthesis of 8-(hydroxymethyl)-3-methyl-1H-pyrazolo[1,5,4-de]quinoxaline-2(3H)-one

**9a-4** (35 mg, 0.130 mmol), (tributyltin)methanol (83 mg, 0.26 mmol) and x-phos Pd G2 (10 mg, 0.013 mmol) in dioxane (10 mL) was stirred at 110°C for 4 h under nitrogen. The reaction solution was concentrated and purified by silica gel column (methanol: DCM = 1:10) to obtain **9a** (25 mg, 0.11 mmol) as a white solid with a yield of 83%. LCMS (ESI): m/z 216.0 [M-H]⁻.

### Intermediate 10a: 3-ethyl-8-(hydroxymethyl)-1H-pyrazolo[1,5,4-de]quinoxaline-2(3H)-one

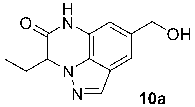

The synthesis method is the same as that of intermediate **9a,** except that tert-butyl 2-bromobutyrate was used as the starting material. LCMS (ESI): m/z 232.2 [M+H]⁺; RT= 1.11 min (3.00 min).

### Intermediate 11a: 8-(hydroxymethyl)-1H-pyrazolo[1,5,4-de]quinoxaline-2(3H)-one

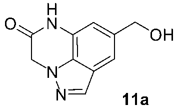

The synthesis method is the same as that of intermediate **9a,** except that tert-butyl bromoacetate was used as the starting material. LCMS (ESI): m/z 202.1 [M-H]⁻; RT= 0.89 min (3.00 min).

### Intermediate 12a: 9-fluoro-8-(hydroxymethyl)-3-methyl-1H-pyrazolo[1,5,4-de]quinoxaline-2(3H)-one

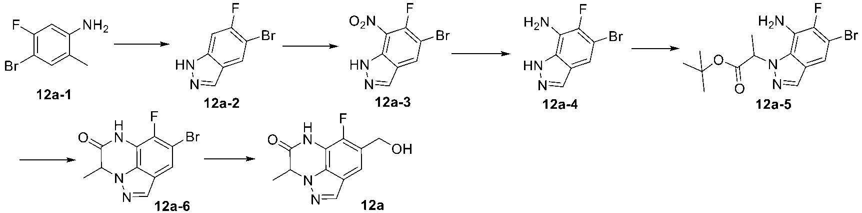

### Step 1: synthesis of 5-bromo-6-fluoro-1H-indazole

**12a-1** (5 g, 24.6 mmol), sodium nitrite (1.7 g, 24.6 mmol) and acetic acid (100 mL) in water (2 mL) was reacted at 50°C for 2 h. The reaction solution was slowly added dropwise to ice water, filtered. The filter cake was washed with water, dried to obtain the product **12a-2** (3.8 g, brown solid), yield:72%. LCMS (ESI): m/z 213.0 [M-H]⁻; RT=1.44 min (3.0 min).

### Step 2: synthesis of 5-bromo-6-fluoro-7-nitro-1H-indazole

**12a-2** (2 g, 9.3 mmol) in concentrated sulfuric acid (20 mL) was added with potassium nitrate (944 mg, 9.3 mmol) in batches under ice bath. The mixture was stirred for 2 h, slowly added dropwise to ice water, and extracted with EA (50 mL). The EA phase was washed with water (30 mL), dried, and concentrated. The residue was purified by silica gel column (PE: EA = 15:1) to obtain **12a-3** (800 mg, yellow solid), yield: 33%. LCMS (ESI): m/z 258.0 [M-H]⁻; RT=1.44 min (3.0 min).

### Step 3: synthesis of 5-bromo-6-fluoro-1H-indazole-7-amine

**12a-3** (1.5 g, 5.8 mmol), iron powder (1.9 g, 34.7 mmol), and ammonium chloride (1.8 g, 34.7 mmol) in ethanol (30 mL)/water (10 mL) was reacted at 60°C for 2 h. The mixture was filtered. The filtrate was added with water (30 mL), extracted with EA (50 mL), dried, and concentrated to obtain the crude product **12a-4** (1 g, brown solid), with a yield of 77%. LCMS (ESI): m/z 230.0 [M+H]⁺; RT=1.38 min (3.0 min).

### Step 4: synthesis of tert-butyl 2-(7-amino-5-bromo-6-fluoro-1H-indazol-1-yl)propanoate

**12a-4** (1.5 g, 6.5 mmol), potassium carbonate (1.8 g, 13.0 mmol), and tert-butyl 2-bromopropanoate (2.0 g, 9.8 mmol) in DMF (40 mL) was reacted at 60°C for 6 h. After cooling, water (30 mL) was added, and EA (50 mL) was used for extraction. EA phase was dried and concentrated to obtain the crude product **12a-5** (1 g, brown solid). LCMS (ESI): m/z 302.0 [M-56+H]⁺; RT=1.78 min (3.0 min).

### Step 5: synthesis of 8-bromo-9-fluoro-3-methyl-1H-pyrazolo[1,5,4-de]quinoxaline-2(3H)-one

**12a-5** (1 g, 2.8 mmol) and HCl/methanol (4 M, 5 mL) were added to methanol (10 mL). The mixture was stirred at 50°C for 2 h, concentrated. The residue was purified by reverse phase preparative column (2%~60% ACN (1‰ NH₄HCO₃)) to obtain crude **12a-6** (140 mg, brown solid), yield: 18%. LCMS (ESI): m/z 282.0 [M-H]⁻; RT=1.41 min (3.0 min).

### Step 6: synthesis of 9-fluoro-8-(hydroxymethyl)-3-methyl-1H-pyrazolo[1,5,4-de]quinoxaline-2(3H)-one

**12a-6** (140 mg, 0.49 mmol), (tributyltin)methanol (327 mg, 0.99 mmol), x-Phos Pd G2 (38 mg, 0.049 mmol) in dioxane (10 mL) was reacted at 100 °C for 3 h under nitrogen. After concentrated, the residue was purified by silica gel column (DCM: Me=20:1) to obtain **12a** (70 mg, brown solid), yield: 60%. LCMS (ESI): m/z 234.1 [M-H]⁻; RT = 1.04 min (3.00 min).

### Intermediate 13a: 6-(piperazin-1-yl) nicotinic acid cyanide hydrochloride

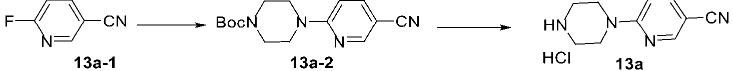

### Step 1: synthesis of tert-butyl 4-(5-cyanopyridin-2-yl)piperazine-1-carboxylate

**13a-1** (500 mg, 4.09 mmol) and 1-tert-butyloxycarbonylpiperazine (915.2 mg, 4.9 mmol) in acetonitrile (10 mL) was added DIEA (1.36 mL, 8.19 mmol). The mixture was stirred at 65°C under nitrogen for 16 h. The solvent was removed and the residue was purified by column chromatography (PE:EA=5:1) to obtain **13a-2** (1.10 g, yellow solid), yield: 93.22%, LCMS (ESI): m/z 289.2 [M+H]⁺; RT=1.728 min (2.50 min).

### Step 2: synthesis of 6-(piperazine-1-yl)nicotinic acid cyanide hydrochloride

**13a-2** (1.10 g, 3.81 mmol) in DCM (15 mL) was added HCl/dioxane hydrochloride (1.5 mL) under ice bath. The mixture was stirred at rt for 2 h and concentrate to obtain **13a** (850 mg, yellow oil), LCMS (ESI): m/z 189.2 [M+H]⁺; RT=0.352 min. ¹H NMR (400 MHz, DMSO-d₆): δ 9.54 (s, 2H), 8.55 (d, J=2.4 Hz, 1H), 7.97-7.94 (m, 1H), 7.03 (d, J=9.2 Hz, 1H), 3.93-3.91 (m, 4H), 3.16 (s, 4H).

### Intermediate 14a: 4-(Hydroxymethyl)-8-methyl-6H-2,2a,6,8-tetraazaacenaphthene-7(8H)-one

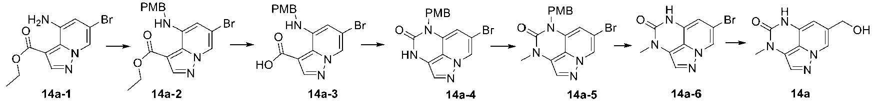

### Step 1: synthesis of 6-bromo-4-((4-methoxybenzyl)amino)pyrazolo[1,5-a]pyridine-3-carboxylic acid ethyl ester

The mixture of **14a-1** (2.0 g, 7.06 mmol), p-methoxybenzaldehyde (1.44 g, 10.6 mmol) and sodium acetate borohydride (6.0 g, 28.24 mmol) in 100 mL of 1,2-dichloroethane was heated to 80°C under argon and stirred overnight. Cooled to rt, the mixture was concentrated under reduced pressure, diluted with water (100 mL) and EA (200 mL*3). EA phase was dried with anhydrous sodium sulfate, filtered, and concentrated. The residue was purified by column (PE:EA =10:1) to obtain compound **14a-2** (2.0 g, white solid), with a yield of 70%. LCMS (ESI): m/z 404.1 [M+H]⁺.

### Step 2: synthesis of 6-bromo-4-((4-methoxybenzyl)amino)pyrazolo[1,5-a]pyridine-3-carboxylic acid

**14a-2** (4.0 g, 9.92 mmol) in tetrahydrofuran (20 mL)/methanol (20 mL)/ distilled water (10 mL) was added sodium hydroxide (2.0 g, 50.0 mmol). The mixture was heated to 60°C for 2h, cooled to rt and concentrated to remove most of the organic solvent, then adjust to pH=5 with 1M dilute hydrochloric acid. Filter the precipitated white solid, and dry in vacuo to obtain **14a-3** (2.5 g, 6.67 mmol), yield: 67%. LCMS (ESI): m/z 274.2 [M+H]⁺.

### Step 3: synthesis of 4-bromo-6-(4-methoxybenzyl)-6H-2,2a,6,8-tetraazaanthracen-7(8H)-one

**14a-3** (500 mg, 1.33 mmol), triethylamine (400 mg, 4.0 mmol) in DMF (10 mL) was added diphenylphosphoryl azide (550 mg, 2.0 mmol) at 0°C. The mixture was stirred for 4 h and then heat to 100°C and stirred overnight. Cooled to rt, the mixture was slowly added dropwise to distilled water. Filter the precipitated gray solid and dry to obtain crude compound **14a-4** (400 mg, white solid), yield: 81%. LCMS (ESI): m/z 373.0 [M+H]⁺.

### Step 4: synthesis of 4-bromo-6-(4-methoxybenzyl)-8-methyl-6H-2,2a,6,8-tetraazaanthracen-7(8H)-one

**14a-4** (500 mg, 1.34 mmol) and potassium carbonate (555 mg, 4.02 mmol) in DMF (10 mL) was added iodomethane (286 mg, 2.01 mmol). The mixture was heated to 60°C and stirred for 4 h. The mixture was cooled to rt and diluted with water (50 mL) and EA (50 mL*3). The EA phase was washed with brine and concentrated under reduced pressure. The residue was purified by silica gel column (PE:EA=1:1) to obtain **14a-5,** 400 mg, yield:77%. LCMS (ESI): m/z 387.0 [M+H]⁺.

### Step 5: synthesis of 4-bromo-8-methyl-6H-2,2a,6,8-tetraazaanthracene-7(8H)-one

The mixture of **14a-5** (400 mg, 1.04 mmol) in TFA (5 mL) was stirred at rt for 4 h, and slowly added dropwise to distilled water. The precipitated gray solid was filtered and dried to obtain **14a-6** (250 mg, white solid) with a yield of 90%, LCMS (ESI): m/z 267.1 [M+H]⁺.

### Step 6: synthesis of 4-(Hydroxymethyl)-8-methyl-6H-2,2a,6,8-tetraazaacenaphthene-7(8H)-one

The synthesis method was the same as step 3 of intermediate **9a,** LCMS (ESI): m/z 219.1 [M+H]⁺.

### Intermediate 15a: 8-(Hydroxymethyl)-3,3-dimethyl-1H-pyrazolo[1,5,4-de]quinoxaline-2(3H)-one

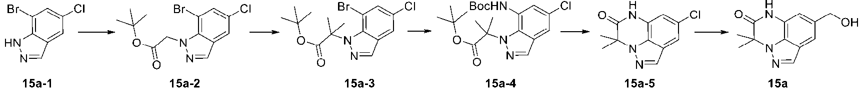

### Step 1: synthesis of tert-butyl (2-(7-bromo-5-chloro-1H-indazol-1-yl)acetate

**15a-1** (2 g, 8.7 mmol), tert-butyl 2-hydroxyacetate (1.72 g, 13.0 mmol), triphenylphosphine (4.1 g, 15.65 mmol) in toluene (40 mL) was added DIAD (3.16 g, 15.65 mmol) at 0°C under nitrogen. The mixture was stirred at rt for 2 h, concentrated and purified by silica gel column (EA:PE=1:4) to obtain product **15a-2** (1.2 g), yield: 40%. LCMS (ESI): m/z 288.9/300.9 [M-55]⁻.

### Step 2: synthesis of tert-butyl 2-(7-bromo-5-chloro-1H-indazol-1-yl)-2-methylpropanoate

**15a-2** (1.2 g, 3.478 mmol) in THF (20 mL) was added LiHMDS (13.91 mmol, 1M/THF) slowly at -78°C in a dry ice acetone bath. After stirred at -78°C for 30 min, iodomethane (1.98 g, 13.91 mmol) was added dropwise to the reaction solution. The temperature was naturally raised to -10°C after continued stirring and quenched by adding saturated aqueous ammonium chloride solution, extracted with ethyl acetate. The EA phase was washed with saturated brine, dried over anhydrous sodium sulfate, filtered, concentrated, and purified by silica gel column (EA:PE=1:5) to obtain **15a-3** (1.1 g, 2.95mmol), yield: 85%. LCMS (ESI): m/z 316.9/318.9 [M-55]-.

### Step 3: synthesis of tert-butyl 2-(7-((tert-butyloxycarbonyl)amino)-5-chloro-1H-indazol-1-yl)-2-methylpropanoate

**15a-3** (1 g, 2.68 mmol), tert-butyl carbamate (3.14 g, 26.8 mmol), Pd₂(dba)₃ (245 mg, 0.268 mmol), 4,5-bis(diphenylphosphino)-9,9-dimethyloxanthene (310 mg, 0.536 mmol) and cesium carbonate (2.61 g, 8.04 mmol) in dioxane (20 mL) was refluxed at 110°C for 8 h under nitrogen. The reaction solution was concentrated and purified by silica gel column (EA:PE=1:2) to obtain **15a-4** (600 mg, 1.46 mmol), yield: 54.5%. LCMS (ESI): m/z 354[M-55]⁻.

Step 4: synthesis of 8-chloro-3,3-dimethyl-1*H*-pyrazolo[1,5,4-de]quinoxaline-2(3H)-one **15a-4** (600 mg, 1.46 mmol) in methanol (10 mL) was added HCl/1,4-dioxane (4 mL). The mixture was stirred at rt for 2 h, concentrated and dried under vacuum to obtain **15a-5** (320 mg, 1.36 mmol), with a yield of 93.2%. LCMS (ESI): m/z 234.1 [M+H]⁺.

### Step 5: synthesis of 8-(hydroxymethyl)-3,3-dimethyl-1H-pyrazolo[1,5,4-de]quinoxaline-2(3H)-one

Refer to the synthesis method of step 3 of intermediate **9a,** LCMS (ESI): m/z 232.1 [M+H]⁺.

### Intermediate 16a: 8'-(hydroxymethyl)spiro[cyclopropane-1,3'-pyrazolo[1,5,4-de]quinoxaline]-2'(1'H)-one

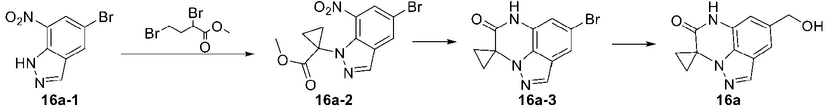

### Step 1: synthesis of 1-(5-bromo-7-nitro-1H-indazol-1-yl)cyclopropane-1-carboxylic acid methyl ester

**16a-1** (500 mg, 2.07 mmol), methyl 2,4-dibromobutyrate (809 mg, 3.11 mmol), Cesium carbonate (2.02 g, 6.21 mmol) in anhydrous DMF was stirred at rt overnight. The reactants were extracted with water (100 mL) and EA (100 mL). The organic phase was washed with saturated brine, dried over anhydrous sodium sulfate, filtered, concentrated, and purified by a silica gel column (PE:EA=10:1) to obtain **16a-2** (80 mg, yellow solid), yield: 12%. LCMS (ESI): m/z 304.1 [M+H]⁺.

### Step 2: synthesis of 8'-bromospiro[cyclopropane-1,3'-pyrazolo[1,5,4-de]quinoxaline]-2'(1'H)-one

**16a-2** (80 mg, 0.23 mmol) and anhydrous acetic acid (10 mL) were heated to 70°C and stirred for 2h. After cooled to rt, the mixture was concentrated. The residue was purified by silica gel column (dichloromethane: methanol = 10:1) to obtain **16a-3** (50 mg, 0.18 mmol, 90% yield). LCMS (ESI): m/z 278.0 [M-H]-.

### Step 3: synthesis of 8'-(hydroxymethyl)spiro[cyclopropane-1,3'-pyrazolo[1,5,4-de]quinoxaline]-2'(1'H)-one

Same synthesis method as step 3 of intermediate **9a.** LCMS (ESI): m/z 230.1 [M+H]⁺.

### Intermediate 17a: 9'-fluoro-8'-(hydroxymethyl)spiro[cyclopropane-1,3'-pyrazolo[1,5,4-de]quinoxaline]-2'(1'H)-one

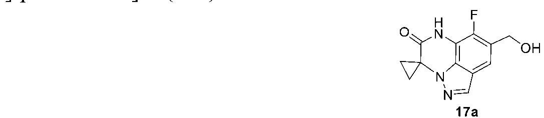

Refer to the synthesis method of intermediate **16a.** LCMS (ESI): m/z 248.1 [M+H]⁺.

### Intermediate 18a: 3-cyclopropyl-8-(hydroxymethyl)-1H-pyrazolo[1,5,4-de]quinoxaline-2(3H)-one

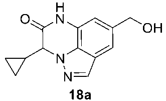

Refer to the synthesis method of intermediate **16a.** LCMS (ESI): m/z 244.1 [M+H]⁺.

### Intermediate 19a: 4-cyclopropyl-8-(hydroxymethyl)-4H-imidazo[1,5,4-de]quinoxalin-5(6H)-one

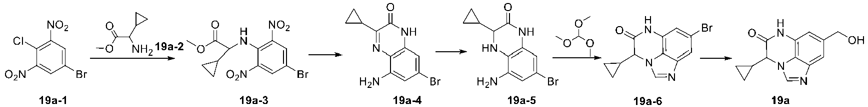

### Step 1: synthesis of methyl 2-[(4-bromo-2,6-dinitrophenyl)amino]-2-cyclopropylacetate

**19a-1** (3.7 g, 0.0131 mol), **19a-2** (2.39 g, 0.0144 mol) and DIEA (5.08 g, 0.0393 mol) in DMF (20 mL) was stirred at 40°C overnight. The reaction solution was added with water and extracted with EA. The organic phase was washed with saturated brine, dried over anhydrous sodium sulfate, filtered, and the filtrate was concentrated and purified by silica gel column (PE:EA=10:1) to obtain **19a-3** (3.3 g, yield: 60.3%, yellow solid). LCMS (ESI): m/z 374.0 [M+H]⁺.

### Step 2: synthesis of 5-amino-7-bromo-3-cyclopropyl-1H-quinoxaline-2-one

**19a-3** (800 mg, 2.1382 mmol), NH₄Cl (1.143 g, 21.381 mmol), and Fe powder (1.194 g, 21.381 mmol) were added to MeOH (50 mL) and H₂O (10 mL). The reaction solution was stirred at 80°C for 16 h and filtered. The solid was washed with methanol. The filtrate was concentrated and diluted with EA, alkalized with saturated sodium bicarbonate aqueous solution, separated. The aqueous phase was extracted with EA. The EA phase was washed with saturated brine, dried over anhydrous sodium sulfate, filtered, and the filtrate was concentrated to obtain crude product **19a-4** (670 mg, yield: 100%, brown solid). LCMS (ESI): m/z 280.0 [M+H]⁺.

### Step 3: synthesis of 5-amino-7-bromo-3-cyclopropyl-3,4-dihydro-1H-quinoxaline-2-one

**19a-4** (670 mg, 2.3918 mmol), NH₄Cl (1.279 g, 21.918 mmol) and Fe (1.564 g, 21.918 mmol) were added to MeOH (50 mL) and H₂O (10 mL). The solid was washed with methanol. The filtrate was concentrated and diluted with EA, alkalized with saturated sodium bicarbonate aqueous solution, separated. The aqueous phase was extracted with EA. The EA phase was washed with saturated brine, dried over anhydrous sodium sulfate, filtered, and the filtrate was concentrated to obtain crude **19a-5** (600 mg, yield: 80%, brown solid). LCMS (ESI): m/z 282.0 [M+H]⁺. ¹H NMR (400 MHz, DMSO-d₆) δ 10.12 (s, 1H), 6.37 (d, J=2.2 Hz, 1H), 6.23 (d, J=2.2 Hz, 1H), 5.13-5.10 (m, 3H), 3.14 (dd, J=2.0, 8.0 Hz, 1H), 1.06-0.88 (m, 1H), 0.51-0.15 (m, 4H).

### Step 4: synthesis of 8-bromo-4-cyclopropyl-4H-imidazo[1,5,4-de]quinoxaline-5(6H)-one

**19a-5** (2.2 g, 2.1266 mmol) in trimethyl orthoformate (20 mL) was added formic acid (0.5 mL). The reaction solution was stirred at 100°C for 2 h, concentrated to obtain a crude product, which was then slurred through EA/PE (1/10, 10 mL) to obtain product **19a-6** (1.8 g, yield: 70.5%, yellow solid), LCMS (ESI): m/z 292.0 [M+H]⁺. ¹H NMR (400 MHz, DMSO-d₆): δ 11.01 (s, 1H), 8.40 (s, 1H), 7.46 (d, J=1.2 Hz, 1H), 6.73 (t, J=1.2 Hz, 1H), 4.69 (d, J=9.0 Hz, 1H), 1.60-1.23 (m, 1H), 0.81-0.41 (m, 4H).

### Step 5: synthesis of 4-cyclopropyl-8-(hydroxymethyl)-4H-imidazo[1,5,4-de]quinoxaline-5(6H)-one

Refer to the synthesis method of intermediate **9a** step 3. LCMS (ESI): m/z 244.2 [M+H]⁺.

### Intermediate 20a: 8-(hydroxymethyl)-4-methyl-6-hydro-2H,4H-oxazolo[3,4,5-de]quinoxaline-2,5-dione

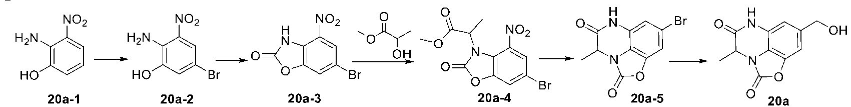

### Step 1: synthesis of 2-amino-5-bromo-3-nitrophenol

**20a-1** (2.5 g, 16.23 mmol) in 1,4-dioxane/acetic acid (20 mL/10 mL) was added bromine (2.49 g, 24.35 mmol) slowly under ice bath. The mixture was stirred at rt for 2 h, and quenched with saturated sodium sulfite aqueous solution, then extracted with EA. The EA phase was dried over anhydrous sodium sulfate. After concentration, the crude product was purified by silica gel column (EA:PE=1:2) to obtain product **20a-2** (2.5 g, 10.7 mmol), yield: 66%. LCMS (ESI): m/z 231/233 [M-H]⁻.

### Step 2: synthesis of 6-bromo-4-nitrobenzo[d]oxazol-2(3H)-one

**20a-2** (1 g, 4.31 mmol) and N,N'-carbonyldiimidazole (1.05 g, 6.47 mmol) in THF (20 mL) was stirred at 70°C for 2h. The reaction solution was concentrated. Water (30 mL) was added to the residue, and the mixture was extracted with EA. The organic phases were combined, dried over anhydrous sodium sulfate, and concentrated. The crude product was purified by silica gel column (EA:PE=1:4) to obtain product **20a-3** (800 mg, 3.09 mmol), yield: 71.6%. LCMS (ESI): m/z 257/259 [M-H]⁻.

### Step 3: synthesis of methyl 2-(6-bromo-4-nitro-2-oxobenzo[d]oxazol-3(2H)-yl)propanoate

**20a-3** (500 mg, 1.94 mmol), methyl 2-hydroxypropanoate (302 mg, 2.91 mmol) and triphenylphosphine (914 mg, 3.49 mmol) in toluene (40 mL) was added to DIAD (705 mg, 3.49 mmol) dropwise under ice bath and nitrogen protection. The mixture was naturally heated to rt and stirred for 4 h. The reaction solution was concentrated and purified by silica gel column (EA:PE=1:4) to obtain product **20a-4** (311 mg, 0.901 mmol), yellow solid, yield: 46%. LCMS (ESI): m/z 345/347 [M+H]⁺.

### Step 4: synthesis of 8-bromo-4-methyl-6-hydrogen-2H,4H-oxazolo[3,4,5-de]quinoxaline-2,5-dione

**20a-4** (341 mg, 0.991 mmol) and iron powder (444 mg, 7.93 mmol) were added to acetic acid (10 mL). The reaction solution was stirred at 70°C for 2 h, concentrated. The residue was purified by silica gel column (methanol: DCM = 1:10) to obtain **20a-5** (195 mg, 0.689 mmol), white solid, yield: 69.5%. LCMS (ESI): m/z 281/283 [M-H]-.

### Step 5: synthesis of 8-(hydroxymethyl)-4-methyl-6-hydrogen-2H,4H-oxazolo[3,4,5-de]quinoxaline-2,5-dione

**20a-5** (100 mg, 0.353 mmol), (tributyltin)methanol (175 mg, 0.530 mmol) and x-phos Pd G2 (28 mg, 0.0353 mmol) in dioxane (15 mL) was stirred at 110°C for 8 h under nitrogen. The reaction solution was concentrated and purified by silica gel column to obtain **20a** (68 mg, 0.289 mmol) as a white solid with a yield of 82%. LCMS (ESI): m/z 235.0 [M+H]⁺.

### Intermediate 21a: 8-(Hydroxymethyl)-4-methyl-4H-imidazo[1,5,4-de]quinoxaline-2,5(1H,6H)-dione

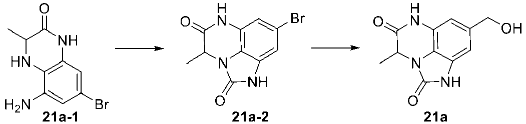

### Step 1: synthesis of 8-bromo-4-methyl-4H-imidazo[1,5,4-de]quinoxaline-2,5(1H,6H)-dione

**21a-1** (80 mg, 0.189 mmol) in DCM (10 mL) was added tri-phosgene (28 mg, 0.094 mmol) and pyridine (45 mg, 0.567 mmol). The mixture was stirred at rt for 2 h, concentrated. The residue was purified by column chromatography (DCM: methanol = 20:1) to obtain **21a-2** (13.5 mg, white solid), yield:16%. LCMS (ESI): m/z 281.9 [M+H]⁺.

### Step 2: synthesis of 8-(hydroxymethyl)-4-methyl-4H-imidazo[1,5,4-de]quinoxaline-2,5(1H,6H)-dione

Refer to the synthesis method of intermediate **9a** step 3. LCMS (ESI): m/z 234.0 [M+H]⁺.

Intermediates **22a-40a:** Refer to the synthesis method of intermediate **14a,** except that 14a-1 or iodomethane was replaced with the raw material intermediates in Table 1 below.

**Table 1**

| Inter media te | structure | name | LCMS (ESI): m/z [M+H]⁺ | Raw material |
|---|---|---|---|---|
| 22a | 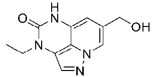 | 4-(hydroxymethyl)-8-ethyl-6*H*-2,2a,6,8-tetraazaacenaphthene -7(8*H*)-one | 281.0 | Ethyl iodide replaces methyl iodide |
| 23a | 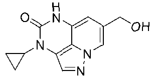 | 4-(hydroxymethyl)-8-cyclopropyl-6*H-*2,2a,6,8-tetraazaacenaphthene -7(8*H*)-one | 293.1 | Cyclopropylboronic acid reacts with 16a-4 in the presence of copper acetate, 2,2'-bipyridine, and sodium carbonate |
| 24a | 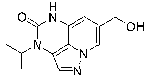 | 4-(hydroxymethyl)-8-isopropyl-6*H-*2,2a,6,8-tetraazaacenaphthene -7(8*H*)-one | 295.1 | 2-bromopropane replaces methyl iodide |
| 25a | 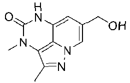 | 4-(hydroxymethyl)-1,8-dimethyl-6*H-*2,2a,6,8-tetraazaacenaphthene -7(8*H*)-one | 280.2 | 4-Amino-6-bromo-2-methylpyrazolo[1,5-a]pyridine-3-carboxylic acid methyl ester instead of 14a-1 |
| 26a | 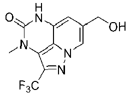 | 4-(hydroxymethyl)-8-methyl-1-(trifluoromethyl)-6*H*-2,2a,6,8-tetraazaacenaphthene -7(8*H*)-one | 335.0 | 4-Amino-6-bromo-2-(trifluoromethyl)pyraz olo[1,5-a]pyridine-3-carboxylic acid ethyl ester was used as 14a-1 |
| 27a | 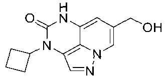 | 4-(hydroxymethyl)-8-cyclobutyl-6*H-*2,2a,6,8-tetraazaacenaphthene -7(8*H*)-one | 307.1 | Bromocyclobutane instead of methyl iodide |
| 28a | 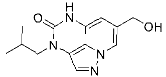 | 4-(hydroxymethyl)-8-isobutyl-6*H-*2,2a,6,8-tetraazaacenaphthene -7(8*H*)-one | 261.2 | 1-Bromo-2-methylpropane instead of methyl iodide |
| 29a | 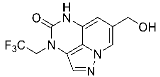 | 4-(hydroxymethyl)-8-trifluoroethyl-6*H-*2,2a,6,8-tetraazaacenaphthene -7(8*H*)-one | 287.0 | 1,1,1-Trifluoro-2-iodoethane instead of methyl iodide |
| 30a | 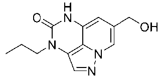 | 4-(hydroxymethyl)-8-propyl-6*H-*2,2a,6,8-tetraazaacenaphthene -7(8*H*)-one | 247.1 | Propane bromoacetate instead of methyl iodide |
| 31a | 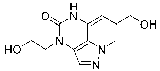 | 8-(2-hydroxyethyl)-4-(hydroxymethyl)-*6H-* 2,2a,6,8-tetraazaacenaphthene -7(8*H*)-one | 249.1 | (2-Bromoethoxy)-tert-butyldimethylsilane |
| 32a | 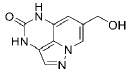 | 4-(hydroxymethyl)-6*H*-2,2a,6,8-tetraazaacenaphthene -7(8*H*)-one | 205.1 | 14a-4 was directly deprotected from PMB |
| 33a | 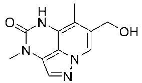 | 4-(hydroxymethyl)-5,8-dimethyl-6*H-*2,2a,6,8-tetraazaacenaphthene -7(8*H*)-one | 233.1 | 4-amino-6-bromo-5-methylpyrazolo[1,5-a]pyridine-3-carboxylic acid ethyl ester replaces 14a-1 |
| 34a | 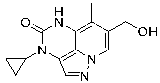 | 8-cyclopropyl-4-(hydroxymethyl)-5-methyl-6*H*-2,2a,6,8-tetraazaacenaphthene -7(8*H*)-one | 259.1 | 4-amino-6-bromo-5-methylpyrazolo[1,5-a]pyridine-3-carboxylic acid ethyl ester replaces 14a-1 |
| 35a | 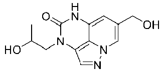 | 4-(hydroxymethyl)-8-(2-hydroxypropyl)-6*H-*2,2a,6,8-tetraazaacenaphthene -7(8*H*)-one | 263.1 | 1-bromopropan-2-ol replaces iodomethane |
| 36a | 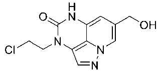 | 8-(2-chloroethyl)-4-(hydroxymethyl)-6*H*-2,2a,6,8-tetraazaacenaphthene -7(8*H*)-one | 315.0 | 1-Bromo-2-chloroethane instead of methyl iodide |
| 37a | 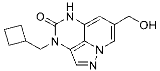 | 8-(cyclobutylmethyl)-4-(hydroxymethyl)-6*H*-2,2a,6,8-tetraazaacenaphthene -7(8*H*)-one | 273.1 | (Bromomethyl)cyclobu tane instead of methyl iodide |
| 38a | 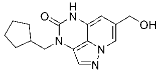 | 8-(cyclopentylmethyl)-4-(hydroxymethyl)-6*H*-2,2a,6,8-tetraazaacenaphthene -7(8*H*)-one | 287.1 | (Bromomethyl)cyclope ntane instead of methyl iodide |
| 39a | 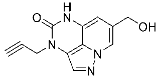 | 4-(hydroxymethyl)-8-(prop-2-yn-1-yl)-6*H*-2,2a,6,8-tetraazaacenaphthene -7(8*H*)-one | 243.1 | 3-Bromopropyne instead of methyl iodide |
| 40a | 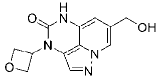 | 4-(hydroxymethyl)-8-(oxetane-3-yl)-6*H-*2,2a,6,8-tetraazaacenaphthene -7(8*H*)-one | 261.2 | 3-Iodooxetane instead of methyl iodide |

### Intermediate 41a: 4-cyclobutyl-8-(hydroxymethyl)-4H-imidazo[1,5,4-de]quinoxaline-5(6H)-one

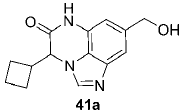

Refer to the intermediate **19a,** except that 2-amino-2-cyclobutylacetic acid methyl ester was used as the starting material. LCMS (ESI): m/z 258.1 [M+H]⁺.

### Synthesis of compound 1

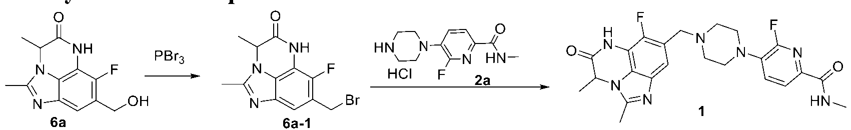

**6a** (15 mg, 0.06 mmol) in DCM (8 mL) was added phosphorus tribromide (0.5 mL) dropwise at rt. The mixture was stirred for 2 h, concentrated and dried to obtain crude product **6a-1. 6a-1** was dissolved in acetonitrile (5 mL), then DIEA (23 mg, 0.18 mmol) and compound **2a** (16 mg, 0.06 mmol) were added in sequence. The mixture was stirred at 80°C for 2 h, concentrated under reduced pressure. The obtained crude product was dissolved in DMSO (3 mL) and purified by reverse phase column (acetonitrile: water (1%o NH₄HCO₃) = 0%-80%) to obtain compound 1 (10 mg, 0.0213 mmol) as a white solid, yield:35.4%. LCMS (ESI): m/z 470.3 [M+H]⁺; RT= 1.24 min (3.00 min). ¹H NMR (400 MHz, DMSO-d₆) δ 11.19 (s, 1H), 8.40 (d, J=4.8 Hz, 1H), 7.85 (t, J=7.6 Hz, 1H), 7.61-7.51 (m, 1H), 7.10 (d, J=4.8 Hz, 1H), 5.33 (q, J=6.8 Hz, 1H), 3.64 (s, 2H), 3.15 (s, 4H), 2.77 (t, J=3.8 Hz, 3H), 2.58 (s, 4H), 2.53 (s, 3H), 1.65 (d, J=7.0 Hz, 3H).

### Synthesis of compounds 4-9 and 11-82

According to the synthesis of compound **1,** intermediates **10a** and **2a** were replaced with the intermediates in Table 2 below separately to synthesize compounds **4-9** and **11-82.**

**Table 2**

| Co mp d | LCMS (ESI): m/z [M+H]⁺ | ¹H NMR (400 MHz, DMSO-*d₆*) δ | interm ediate |
|---|---|---|---|
| 4 | 438.3 | 11.04 (s, 1H), 8.40 (q, J=4.8 Hz, 1H), 8.03 (s, 1H), 7.84 (dd, J=8.1, 1.3 Hz, 1H), 7.56 (dd, J=10.6, 8.1 Hz, 1H), 7.19 (s, 1H), 6.73 (d, J=0.9 Hz, 1H), 5.34 (q, J=6.9 Hz, 1H), 3.56 (s, 2H), 3.22-3.12 (m, 4H), 2.76 (d, J=4.8 Hz, 3H), 2.55 (m, 4H), 1.75 (d, J=6.9 Hz, 3H). | 9a, 2a |
| 5 | 452.4 | 11.13 (s, 1H), 8.40 (q, J=4.8 Hz, 1H), 8.06 (s, 1H), 7.84 (dd, J=8.1, 1.3 Hz, 1H), 7.56 (dd, J=10.6, 8.1 Hz, 1H), 7.20 (d, J=0.9 Hz, 1H), 6.73 (d, J=1.0 Hz, 1H), 5.40 (t, J=4.1 Hz, 1H), 3.61-3.50 (m, 2H), 3.17-3.15 (m, 4H), 2.76 (d, J=4.8 Hz, 3H), 2.58-2.52 (m, 4H), 2.32-2.20 (m, 2H), 0.61 (t, J=7.3 Hz, 3H). | 10a, 2a |
| 6 | 454.3 | 11.04 (s, 1H), 8.42 (q, *J*=4.8 Hz, 1H), 8.03 (s, 1H), 7.93 (d, *J*=8.2 Hz, 1H), 7.65 (d, *J*=8.2 Hz, 1H), 7.20 (s, 1H), 6.73 (d, *J*=0.9 Hz, 1H), 5.33 (q, *J*=6.9 Hz, 1H), 3.57 (s, 2H), 3.09 (m, 4H), 2.78 (d, *J*=4.8 Hz, 3H), 2.57 (m, 4H), 1.75 (d, *J*=6.9 Hz, 3H). | 9a, 1a |
| 7 | 434.3 | 11.04 (s, 1H), 8.41 (q, *J*=4.8 Hz, 1H), 8.03 (s, 1H), 7.79 (d, *J*=8.2 Hz, 1H), 7.47 (d, *J*=8.3 Hz, 1H), 7.20 (s, 1H), 6.74 (d, *J*=0.9 Hz, 1H), 5.33 (q, *J*=6.9 Hz, 1H), 3.57 (s, 2H), 2.92 (m, 4H), 2.80 (d, *J*=4.9 Hz, 3H), 2.55 (m, 4H), 2.48 (s, 3H), 1.75 (d, *J*=6.9 Hz, 3H). | 9a, 3a |
| 8 | 468.3 | 11.13 (s, 1H), 8.42 (q, *J*=4.8 Hz, 1H), 8.06 (s, 1H), 7.93 (d, *J*=8.1 Hz, 1H), 7.65 (d, *J*=8.2 Hz, 1H), 7.21 (s, 1H), 6.73 (d, *J*=1.0 Hz, 1H), 5.40 (t, *J*=4.0 Hz, 1H), 3.57 (q, *J*=12.8 Hz, 2H), 3.10 (m, 4H), 2.79 (d, *J*=4.8 Hz, 3H), 2.57 (m, 4H), 2.33-2.17 (m, 2H), 0.61 (t, *J*=7.3 Hz, 3H). | 10a, 1a |
| 9 | 448.3 | 11.13 (s, 1H), 8.41 (d, *J=5.0* Hz, 1H), 8.05 (s, 1H), 7.79 (d, J=8.3 Hz, 1H), 7.47 (d, *J*=8.3 Hz, 1H), 7.21 (s, 1H), 6.73 (d, *J*=0.9 Hz, 1H), 5.40 (t, *J*=4.1 Hz, 1H), 3.64-3.50 (m, 2H), 2.93 (m, 4H), 2.80 (d, *J*=4.9 Hz, 3H), 2.55 (m, 4H), 2.48 (s, 3H), 2.32-2.20 (m, 2H), 0.60 (t, *J*=7.3 Hz, 3H). | 10a, 3a |
| 11 | 473.2 | 11.38 (s, 1H), 8.43-8.40 (m, 1H), 8.08 (s, 1H), 7.92 (d, *J*=8.0 Hz, 1H), 7.64 (d, *J*=8.4 Hz, 1H), 7.29 (d, *J*=5.2 Hz, 1H), 5.33 (q, *J*=6.8 Hz, 1H), 3.65 (s, 2H), 3.10-3.09 (m, 4H), 2.78 (d, *J*=5.2 Hz, 3H), 2.60-2.59 (m, 4H), 1.75 (d, *J*=6.8 Hz, 3H). | 12a, 1a |
| 12 | 452.3 | 11.37 (s, 1H), 8.41 (q, *J*=4.8 Hz, 1H), 8.08 (s, 1H), 7.78 (d, *J*=8.4 Hz, 1H), 7.46 (d, *J*=8.4 Hz, 1H), 7.30 (d, *J*=5.0 Hz, 1H), 5.33 (q, *J*=6.8 Hz, 1H), 3.65 (s, 2H), 2.94-2.90 (m, 4H), 2.79 (d, *J*=4.9 Hz, 3H), 2.61-2.58 (m, 4H), 2.48 (s, 3H), 1.75 (d, *J*=7.0 Hz, 3H). | 12a, 3a |
| 16 | 420.3 | 11.07 (s, 1H), 8.42 (d, *J*=4.9 Hz, 1H), 8.01 (s, 1H), 7.79 (d, *J*=8.3 Hz, 1H), 7.47 (d, *J*=8.3 Hz, 1H), 7.20 (s, 1H), 6.74 (s, 1H), 5.20 (s, 2H), 3.59 (s, 2H), 2.94 (m, 4H), 2.80 (d, *J*=4.9 Hz, 3H), 2.56 (m, 4H), 2.49 (s, 3H). | 11a, 3a |
| 17 | 435.2 | 10.00 (s, 1H), 8.40 (d, *J*=4.8 Hz, 1H), 7.84 (d, *J*=7.2 Hz, 1H), 7.73 (s, 1H), 7.56 (dd, *J*=10.6, 8.2 Hz, 1H), 7.40 (s, 1H), 5.76 (s, 1H), 3.34 (s, 2H), 3.16 (s, 4H), 3.12 (s, 3H), 2.76 (d, *J*=4.8 Hz, 3H), 2.53 (d, *J*=12.9 Hz, 4H). | 14a, 2a |
| 19 | 435.2 | 10.01 (s, 1H), 8.41 (d, *J*=4.8 Hz, 1H), 7.79 (d, *J*=8.2 Hz, 1H), 7.74 (s, 1H), 7.47 (d, *J*=8.2 Hz, 1H), 7.40 (s, 1H), 5.77 (s, 1H), 3.36 (s, 2H), 3.12 (s, 3H), 2.93 (s, 4H), 2.80 (d, *J*=4.8 Hz, 3H), 2.56 (s, 4H), 2.48 (s, 3H). | 14a, 3a |
| 25 | 424.2 | 11.06 (s, 1H), 8.39 (d, *J*=4.8 Hz, 1H), 8.00 (s, 1H), 7.84 (dd, *J*=8.0, 0.9 Hz, 1H), 7.55 (dd, *J*=10.6, 8.2 Hz, 1H), 7.19 (s, 1H), 6.73 (s, 1H), 5.19 (s, 2H), 3.56 (s, 2H), 3.15 (s, 4H), 2.76 (d, *J*=4.8 Hz, 3H), 2.54 (s, 4H) | 11a, 2a |
| 26 | 440.2 | 11.07 (s, 1H), 8.42 (q, *J*=4.7 Hz, 1H), 8.01 (s, 1H), 7.93 (d, *J*=8.2 Hz, 1H), 7.65 (d, *J*=8.2 Hz, 1H), 7.19 (s, 1H), 6.73 (s, 1H), 5.19 (s, 2H), 3.58 (s, 2H), 3.10 (s, 4H), 2.78 (d, *J*=4.8 Hz, 3H), 2.55 (s, 4H). | 11a, 3a |
| 27 | 448.3 | 11.04 (s, 1H), 8.41 (t, *J*=4.9 Hz, 1H), 8.05 (s, 1H), 7.79 (d, *J*=8.2 Hz, 1H), 7.47 (d, *J*=8.3 Hz, 1H), 7.20 (s, 1H), 6.74 (s, 1H), 3.57 (s, 2H), 2.93 (m, 4H), 2.80 (d, *J*=4.9 Hz, 3H), 2.57 (m, 4H), 2.48 (s, 3H), 1.73 (s, 6H). | 15a, 3a |
| 30 | 449.2 | 9.97 (s, 1H), 8.42-8.41 (m, 1H), 7.78 (d, *J*=7.8 Hz, 1H), 7.73 (s, 1H), 7.47 (d, *J*=7.8 Hz, 1H), 7.43 (s, 1H), 5.76 (s, 1H), 3.64 (q, *J*=7.2 Hz, 1H), 3.36 (s, 2H), 2.94-2.92 (m, 4H), 2.79 (d, *J*=4.8 Hz, 3H), 2.57-2.55 (m, 4H), 2.48 (s, 3H), 1.16 (t, *J*=6.6 Hz, 3H). | 22a, 3a |
| 33 | 453.1 | 9.98 (s, 1H), 8.40-8.39 (m, 1H), 7.84 (d, *J*=7.2 Hz, 1H), 7.73 (s, 1H), 7.58-7.54 (m, 1H), 7.43 (s, 1H), 5.75 (s, 1H), 3.64 (q, *J*=6.8 Hz, 2H), 3.36 (s, 2H), 3.11-2.98 (m, 4H), 2.79 (d, *J*=4.8 Hz, 3H), 2.57-2.54 (m, 4H), 1.16 (t, *J*=6.8 Hz, 3H). | 22a, 2a |
| 34 | 469.1 | 9.97 (s, 1H), 8.43-8.42 (m, 1H), 7.93 (d, *J*=8.4 Hz, 1H), 7.73 (s, 1H), 7.66 (d, *J*=8.0 Hz, 1H), 7.43 (s, 1H), 5.75 (s, 1H), 3.64 (q, *J*=6.8 Hz, 2H), 3.36 (s, 2H), 3.10-2.98 (m, 4H), 2.79 (d, *J*=4.8 Hz, 3H), 2.57-2.54 (m, 4H), 1.16 (t, *J*=6.8 Hz, 3H). | 22a, la |
| 35 | 388.2 | 11.04 (s, 1H), 8.47 (d, *J*=2.1 Hz, 1H), 8.03 (s, 1H), 7.84 (dd, *J*=9.1, 2.4 Hz, 1H), 7.18 (s, 1H), 6.91 (d, *J*=9.1 Hz, 1H), 6.73 (s, 1H), 5.33 (q, *J*=6.9 Hz, 1H), 3.65-3.63 (m, 4H), 3.53 (s, 2H), 2.45-2.42 (m, 4H), 1.75 (d, *J*=7.0 Hz, 3H). | 9a, 13a |
| 37 | 450.2 | 11.16 (s, 1H), 8.40 (q, *J*=4.5 Hz, 1H), 7.93 (d, *J*=5.4 Hz, 1H), 7.84 (d, *J*=7.8 Hz, 1H), 7.56 (dd, *J*=10.5*,* 8.2 Hz, 1H), 7.17 (s, 1H), 6.71 (s, 1H), 3.55 (s, 2H), 3.16 (s, 4H), 2.77 (d, *J*=4.8 Hz, 3H), 2.55 (s, 4H), 1.75 (dd, *J=8.0,* 4.6 Hz, 2H), 1.60 (dd, *J*=8.0, 4.6 Hz, 2H). | 16a, 2a |
| 38 | 466.1 | 11.17 (s, 1H), 8.43 (q, *J*=4.6 Hz, 1H), 7.93 (dd, *J*=8.0, 3.2 Hz, 2H), 7.66 (d, *J*=8.2 Hz, 1H), 7.18 (s, 1H), 6.71 (s, 1H), 3.57 (s, 2H), 3.10 (s, 4H), 2.79 (d, *J*=4.8 Hz, 3H), 2.56 (d, *J*=15.5 Hz, 4H), 1.75 (dd, *J*=8.0, 4.6 Hz, 2H), 1.60 (dd, *J*=8.0, 4.6 Hz, 2H). | 16a, 1a |
| 39 | 446.3 | 8.41 (q, J=4.8 Hz, 1H), 7.93 (s, 1H), 7.79 (d, J=8.3 Hz, 1H), 7.47 (d, J=8.3 Hz, 1H), 7.18 (s, 1H), 6.72 (s, 1H), 3.57 (s, 2H), 2.93 (s, 4H), 2.80 (d, *J*=4.9 Hz, 3H), 2.55 (d, *J*=12.6 Hz, 4H), 2.49 (s, 3H), 1.74 (dd, *J*=8.0, 4.6 Hz, 2H), 1.60 (dd, *J*=8.1, 4.6 Hz, 2H). | 16a, 3a |
| 40 | 460.3 | 11.17 (s, 1H), 8.46 (q, *J*=4.7 Hz, 1H), 7.93 (d, *J*=2.9 Hz, 1H), 7.82 (d, J=8.2 Hz, 1H), 7.63 (d, *J*=8.3 Hz, 1H), 7.18 (s, 1H), 6.73 (s, 1H), 3.54 (s, 2H), 3.2 (s, 1H), 3.03 (d, *J*=10.6 Hz, 1H), 2.80 (d, *J*=4.9 Hz, 3H), 2.76 (d, *J*=10.2 Hz, 1H), 2.62 (t, *J*=9.3 Hz, 2H), 2.49 (s, 3H), 2.42 (d, *J*=9.8 Hz, 1H), 2.12 (s, 1H), 1.75 (dd, *J*=7.9, 4.5 Hz, 2H), 1.60 (dd, *J*=8.0, 4.6 Hz, 2H), 0.78 (d, *J*=6.1 Hz, 3H). | 16a, 4a |
| 41 | 484.3 | 11.50 (s, 1H), 8.43 (d, *J*=4.8 Hz, 1H), 7.99 (s, 1H), 7.93 (d, *J*=8.2 Hz, 1H), 7.65 (d, *J*=8.2 Hz, 1H), 7.30 (d, *J*=5.2 Hz, 1H), 3.67 (s, 2H), 3.11 (s, 4H), 2.79 (d, *J*=4.8 Hz, 3H), 2.64 (s, 4H), 1.78-1.74 (m, 2H), 1.62-1.59 (m, 2H). | 17a, 1a |
| 42 | 464.3 | 11.48 (s, 1H), 8.41 (d, *J*=4.9 Hz, 1H), 7.98 (s, 1H), 7.78 (d, *J*=8.3 Hz, 1H), 7.46 (d, *J*=8.3 Hz, 1H), 7.28 (d, *J*=5.1 Hz, 1H), 3.64 (s, 2H), 2.93 (s, 4H), 2.80 (d, *J*=4.9 Hz, 3H), 2.61 (s, 4H), 2.48 (s, 3H), 1.75 (dd, *J*=8.0, 4.6 Hz, 2H), 1.60 (dd, *J*=8.1, 4.7 Hz, 2H). | 17a, 3a |
| 51 | 451.3 | 10.90 (s, 1H), 8.41 (q, *J*=4.7 Hz, 1H), 7.79 (d, *J*=8.3 Hz, 1H), 7.47 (d, *J*=8.3 Hz, 1H), 6.93 (s, 1H), 6.68 (s, 1H), 4.87 (q, *J*=7.0 Hz, 1H), 3.53 (s, 2H), 2.93 (m, 4H), 2.80 (d, *J*=4.9 Hz, 3H), 2.55 (m, 4H), 2.48 (s, 3H), 1.67 (d, *J*=7.0 Hz, 3H). | 20a, 3a |
| 52 | 450.2 | 10.82 (s, 1H), 10.69 (s, 1H), 8.41 (q, *J*=4.8 Hz, 1H), 7.79 (d, *J*=8.2 Hz, 1H), 7.47 (d, *J*=8.2 Hz, 1H), 6.59 (d, *J*=8.2 Hz, 1H), 6.48 (d, *J*=10.7 Hz, 1H), 4.78 (q, *J*=6.8 Hz, 1H), 3.49 (s, 3H), 2.92 (s, 4H), 2.80 (d, *J*=4.8 Hz, 3H), 2.53 (d, *J*=6.4 Hz, 1H), 2.48 (s, 3H), 1.61 (d, *J*=7.2 Hz, 3H). | 21a, 3a |
| 53 | 465.3 | 9.96 (s, 1H), 8.40 (d, *J*=4.6 Hz, 1H), 7.84 (d, *J*=7.9 Hz, 1H), 7.73 (s, 1H), 7.60-7.51 (m, 1H), 7.37 (s, 1H), 5.76 (s, 1H), 3.34 (s, 2H), 3.16 (m, 4H), 2.76 (d, *J*=4.5 Hz, 4H), 2.53 (m, 4H), 0.92 (d, *J*=5.7 Hz, 2H), 0.75 (m, 2H). | 23a, 2a |
| 54 | 461.3 | 9.96 (s, 1H), 8.41 (q, *J*=4.6 Hz, 1H), 7.79 (d, *J*=8.3 Hz, 1H), 7.74 (s, 1H), 7.47 (d, *J*=8.3 Hz, 1H), 7.37 (s, 1H), 5.75 *(*d, *J=*11.1 Hz, 1H), 3.36 (s, 2H), 2.93 (m, 4H), 2.80 (m, 4H), 2.55 (m, 4H), 2.48 (s, 3H), 0.92 (q, *J*=7.0 Hz, 2H), 0.79 - 0.70 (m, 2H). | 23a, 3a |
| 55 | 467.3 | 9.95 (s, 1H), 8.42-8.38 (m, 1H), 7.84 (d, *J*=8.0 Hz, 1H), 7.72 (s, 1H), 7.58-7.54 (m, 2H), 5.74 (s, 1H), 4.78 (q, *J*=6.8 Hz, 1H), 3.36 (s, 2H), 3.16-3.14 (m, 4H), 2.76 (d, *J*=4.8 Hz, 3H), 2.54-2.52 (m, 4H), 1.32 (d, *J*=6.8 Hz, 6H). | 24a, 2a |
| 56 | 463.3 | 9.95 (s, 1H), 8.42-8.41 (m, 1H), 7.78 (d, *J*=8.0 Hz, 1H), 7.73 (s, 1H), 7.56 (s, 1H), 7.47 (d, *J*=8.4 Hz, 1H), 5.75 (s, 1H), 4.76 (q, *J=6.8* Hz, 1H), 3.36 (s, 2H), 2.94-2.92 (m, 4H), 2.79 (d, *J*=4.8 Hz, 3H), 2.57-2.55 (m, 4H), 2.48 (s, 3H), 1.32 (d, *J*=6.8 Hz, 6H). | 24a, 3a |
| 57 | 449.4 | 9.88 (s, 1H), 8.46-8.37 (m, 1H), 7.79 (d, *J*=8.2 Hz, 1H), 7.60 (s, 1H), 7.47 (d, *J*=8.3 Hz, 1H), 5.70 (s, 1H), 3.33 (m, 2H), 3.24 (s, 3H), 2.93 (s, 4H), 2.80 (d, *J*=4.9 Hz, 3H), 2.55 (m, 4H), 2.48 (s, 3H), 2.42 (s, 3H). | 25a, 3a |
| 58 | 503.2 | 10.48 (s, 1H), 8.41 (d, *J*=4.9 Hz, 1H), 7.92 (s, 1H), 7.79 (d, *J*=8.2 Hz, 1H), 7.48 (d, *J*=8.3 Hz, 1H), 5.98 (s, 1H), 3.43 (s, 2H), 3.22 (s, 3H), 2.94-2.92 (m, 4H), 2.80 (d, *J*=4.9 Hz, 3H), 2.58-2.56 (m, 4H), 2.49 (s, 3H). | 26a, 3a |
| 59 | 475.3 | 10.01 (s, 1H), 8.41 (d, *J*=4.2 Hz, 1H), 7.79-7.75 (m, 2H), 7.68 (s, 1H), 7.48 (d, *J*=8.4 Hz, 1H), 5.77 (s, 1H), 5.01-4.99 (m, 1H), 3.36 (s, 2H), 2.94-2.92 (m, 4H), 2.80 (d, *J*=5.4 Hz, 3H), 2.68-2.65 (m, 2H), 2.56-2.54 (m, 4H), 2.48 (s, 3H), 2.11-2.07 (m, 2H), 1.85-1,82 (m, 1H), 1.71-1.67 (m, 1H). | 27a, 3a |
| 60 | 477.2 | 9.99 (s, 1H), 8.42 (d, *J*=4.5 Hz, 1H), 7.80 (d, *J=*8.1 Hz, 1H), 7.74 (s, 1H), 7.48 (d, *J*=7.9 Hz, 1H), 7.43 (s, 1H), 5.78 (s, 1H), 3.42 (d, *J*=7.4 Hz, 2H), 3.34 (s, 2H), 2.94 (s, 4H), 2.80 (d, *J*=4.8 Hz, 3H), 2.67 (s, 4H), 2.49 (s, 3H), 2.16 - 2.04 (m, 1H), 0.90 (d, *J*=6.6 Hz, 6H). | 28a, 3a |
| 61 | 503.2 | 10.35 (s, 1H), 8.41 (q, *J*=4.6 Hz, 1H), 7.82 (s, 1H), 7.79 (d, *J*=8.2 Hz, 1H), 7.48 -7.41(m, 2H), 5.88 (s, 1H), 4.52 (q, *J*=9.2 Hz, 2H), 3.39 (s, 2H), 2.94 (m, 4H), 2.80 (d, *J*=4.8 Hz, 3H), 2.63-2.53 (m, 4H), 2.49 (s, 3H) | 29a, 3a |
| 62 | 463.3 | 9.97 (s, 1H), 8.41 (d, *J*=4.9 Hz, 1H), 7.79 (d, *J*=8.2 Hz, 1H), 7.73 (s, 1H), 7.48 (d, *J*=8.3 Hz, 1H), 7.43 (s, 1H), 5.76 (s, 1H), 3.57 (t, *J*=7.1 Hz, 2H), 3.36 (s, 2H), 2.94-2.92 (m, 4H), 2.80 (d, *J*=4.9 Hz, 3H), 2.56-2.54 (m, 4H), 2.49 (s, 3H), 1.63 (dd, *J*=14.4, 7.3 Hz, 2H), 0.90 (t,*J*=7.4 Hz, 3H). | 30a, 3a |
| 63 | 465.3 | 9.97 (s, 1H), 8.42 (q, *J*=4.7 Hz, 1H), 7.79 (d, *J*=8.2 Hz, 1H), 7.72 (s, 1H), 7.48 (d, *J*=8.3 Hz, 1H), 7.36 (s, 1H), 5.75 (s, 1H), 4.80 (s, 1H), 3.64 (dd, *J*=13.5*,* 4.6 Hz, 4H), 3.35 (s, 2H), 2.93-2.90 (m, 4H), 2.80 (d, *J*=4.9 Hz, 3H), 2.56-2.54 (m, 4H), 2.48 (s, 3H). | 31a, 3a |
| 64 | 421.3 | 9.77 (s, 1H), 9.48 (s, 1H), 8.42 (d, *J*=4.9 Hz, 1H), 7.79 (d, *J*=8.2 Hz, 1H), 7.70 (s, 1H), 7.48 (d, *J*=8.3 Hz, 1H), 7.13 (s, 1H), 5.74 (s, 1H), 3.33 (s, 2H), 2.94 (s, 4H), 2.80 (d, *J*=4.9 Hz, 3H), 2.56 (s, 4H), 2.49 (s, 3H). | 32a, 3a |
| 65 | 449.2 | 9.66 (s, 1H), 8.42 (d, *J*=4.0 Hz, 1H), 7.78-7.76 (m, 2H), 7.47 (d, *J*=8.0 Hz, 1H), 7.35 (s, 1H), 3.60 (s, 2H), 3.37 (s, 3H). 3.12 (s, 3H), 2.93 (s, 4H), 2.80 (d, *J*=4.8 Hz, 3H), 2.57 (s, 4H), 2.48 (s, 3H). | 33a, 3a |
| 66 | 475.4 | 9.61 (s, 1H), 8.45-8.37 (m, 1H), 7.80-7.75 (m, 2H), 7.47 (d, *J*=8.3 Hz, 1H), 7.33 (s, 1H), 3.36 (s, 2H), 2.91 (s, 4H), 2.80 (d, *J*=4.8 Hz, 3H), 2.77-2.73 (m, 1H), 2.56 (s, 4H), 2.49 (s, 3H), 2.07 (s, 3H), 0.96-0.90 (m, 2H), 0.77-0.72 (m, 2H). | 34a, 3a |
| 67 | 479.4 | 9.98 (s, 1H), 8.42 (q, *J*=4.7 Hz, 1H), 7.79 (d, *J*=8.2 Hz, 1H), 7.72 (s, 1H), 7.48 (d, *J*=8.3 Hz, 1H), 7.36 (s, 1H), 5.75 (s, 1H), 4.80 (d, *J*=5.0 Hz, 1H), 3.97 (dt, *J*=12.0*,* 6.1 Hz, 1H), 3.50 (d, *J*=6.1 Hz, 2H), 3.39 (m, 2H), 2.95 (m, 4H), 2.80 (d, *J*=4.9 Hz, 3H), 2.60 (m, 4H), 2.49 (s, 3H), 1.27-1.08 (m, 3H). | 35a, 3a |
| 68 | 447.1 | 8.41 (d, *J*=4.8 Hz, 1H), 7.79 (d, *J*=8.3 Hz, 1H), 7.58 (s, 1H), 7.48 (d, *J=* 8.3 Hz, 1H), 7.26 (s, 1H), 5.73 (s, 1H), 4.59 (t, *J*=7.9 Hz, 2H), 3.85 (t, *J=* 7.9 Hz, 2H), 3.30 (s, 2H), 2.94 (s, 4H), 2.80 (d, *J*=4.8 Hz, 3H), 2.56 (s, 4H), 2.48 (s, 3H). | 36a, 3a |
| 69 | 489.4 | 9.98 (s, 1H), 8.43-8.42 (m, 1H), 7.83-7.73 (m, 2H), 7.53-7.45 (m, 2 H), 5.77 (s, 1H), 3.66-3.64 (m, 2H), 3.32 (s, 2H), 2.81-2.79 (m, 4H), 2.77-2.73 (m, 3H), 2.71-2.69 (m, 1H), 2.57-2.52 (m, 7H), 2.20-1.94 (m, 2H), 1.87-1.76 (m, 4H). | 37a, 3a |
| 70 | 503.4 | 9.97 (s, 1H), 8.42 (q, *J*=4.8 Hz, 1H), 7.79 (d, *J*=8.2 Hz, 1H), 7.73 (s, 1H), 7.48 (d, *J*=8.3 Hz, 1H), 7.43 (s, 1H), 5.76 (s, 1H), 3.54 (d, *J=7.6* Hz, 2H), 3.36 (s, 2H), 2.94 (s, 4H), 2.80 (d, *J*=4.9 Hz, 3H), 2.56 (s, 4H), 2.49 (s, 3H), 2.41-2.34 (m, 1H), 1.70-1.57 (m, 4H), 1.53-1.42 (m, 2H), 1.29-1.26 (m, 2H). | 38a, 3a |
| 71 | 461.3 | 8.41 (q, *J*=4.6 Hz, 1H), 7.79 (d, *J*=8.3 Hz, 1H), 7.58 (s, 1H), 7.48 (d, *J*=8.3 Hz, 1H), 7.23 (s, 1H), 5.73 (s, 1H), 5.05-4.93 (m, 1H), 3.98 (t, *J*=8.3 Hz, 1H), 3.47-3.41 (m, 1H), 3.30 (s, 2H), 2.94 (m, 4H), 2.80 (d, *J*=4.9 Hz, 3H), 2.56 (m, 4H), 2.48 (s, 3H), 1.44 (d, *J*=6.2 Hz, 3H) | 35a, 3a |
| 72 | 489.4 | 10.02 (s, 1H), 8.48-8.44 (m, 1H), 7.81 (d, *J*=8.4 Hz, 1H), 7.76 (s, 1H), 7.68 (s, 1H), 7.64 (d, *J*=8.4 Hz, 1H), 5.78 (s, 1H), 5.02-4.98 (m, 1H), 3.26 (s, 2H), 3.04-3.02 (m, 1H), 2.80 (d, *J*=4.8 Hz, 3H), 2.78-2.60 (m, 5H), 2.50-2.49 (m, 4H), 2.41-2.39 (m, 1H), 2.12-2.05 (m, 3H), 1.86-1.83 (m, 1H), 1.70-1.67(m, 1H), 0.78 (d, *J*=6.4 Hz, 3H). | 27a, 5a |
| 73 | 495.2 | 10.02 (s, 1H), 8.43 (q, *J*=4.8 Hz, 1H), 7.93 (d, *J*=8.4 Hz, 1H), 7.76 (s, 1H), 7.68 (s, 1H), 7.66 (d, *J*=7.8 Hz, 1H), 5.77 (s, 1H), 5.02-4.99 (m, 1H), 3.36 (s, 2H), 3.10 (s, 4H), 2.79 (d, *J*=4.8 Hz, 3H), 2.70-2.65 (m, 2H), 2.57 (s, 4H), 2.12-2.07 (m, 2H), 1.85-1.82 m, 1H), 1.71-1.68 (m, 1H). | 27a, 1a |
| 76 | 459.3 | 10.20 (s, 1H), 8.42 (q, *J*=4.8 Hz, 1H), 7.80-7.78 (m, 2H), 7.48 (d, *J*=8.4 Hz, 1H), 7.44 (s, 1H), 5.84 (s, 1H), 4.47 (d, *J*=1.8 Hz, 2H), 3.38 (s, 2H), 3.25 (t, *J*=2.4 Hz, 1H), 2.94 (s, 4H), 2.80 (d, *J*=5.4 Hz, 3H), 2.57 (s, 4H), 2.49 (s, 3H). | 39a, 3a |
| 78 | 474.4 | 10.87 (s, 1H), 8.41 (q, *J*=4.8 Hz, 1H), 8.26 (s, 1H), 7.79 (d, *J*=8.4 Hz, 1H), 7.47 (d, *J*=8.4 Hz, 1H), 7.18 (s, 1H), 6.67 (s, 1H), 5.16 (d, *J*=7.2 Hz, 1H), 3.63-3.55 (m, 2H), 2.93-2.84 (m, 5H), 2.80 (d, *J*=4.8 Hz, 3H), 2.57 (brs, 4H), 2.48 (s, 3H), 2.12-2.02 (m, 1H), 1.97-1.71 (m, 4H), 1.67-1.58 (m, 1H). | 41a, 3a |
| 79 | 489.4 | 9.98 (s, 1H), 8.41 (q, *J*=4.8 Hz, 1H), 7.80-7.77 (m, 2H), 7.67 (s, 1H), 7.46 (d, *J*=8.4 Hz, 1H), 5.77 (s, 1H), 5.00 (p, *J*=9.2 Hz, 1H), 3.80 (d, *J=13.2* Hz, 1H), 3.10-2.98 (m, 3H), 2.80-2.76 (m, 5H), 2.69-2.61 (m, 4H), 2.49 (brs, 3H), 2.35-2.32 (m, 1H), 2.12-2.01 (m, 2H), 1.88-1.65 (m, 2H), 1.16 (d, *J =*5.6 Hz, 3H). | 27a, 4a |
| 80 | 509.3 | 9.98 (s, 1H), 8.43 (q, *J*=4.7 Hz, 1H), 7.93 (d, *J*=8.2 Hz, 1H), 7.77 (s, 1H), 7.68 (s, 1H), 7.65 (d, *J*=8.2 Hz, 1H), 5.76 (s, 1H), 5.03-4.97 (m, 1H), 3.79 (d, *J=*13.2 Hz, 1H), 3.25-3.32 (m, 2H), 3.09 (d, *J=*13.2 Hz, 1H), 2.88 (t, *J*=9.6 Hz, 1H), 2.79-2.74 (m, 5H), 2.67-2.63 (m, 3H), 2.33 (t, *J*=9.0 Hz, 1H), 2.11-2.07 (m, 2H), 1.87-1.82 (m, 1H), 1.73-1.65(m, 1H), 1.16 (d, *J*=6.2 Hz, 3H). | 27a, 6a |
| 81 | 494.3 | 10.87 (s, 1H), 8.43 (q, *J*=4.8 Hz, 1H), 8.26 (s, 1H), 7.93 (d, *J*=8.4 Hz, 1H), 7.66 (d, J=8.4 Hz, 1H), 7.18 (s, 1H), 6.66 (s, 1H), 5.16 (d, *J=*7.2 Hz, 1H), 3.63-3.56 (m, 2H), 3.10 (s, 4H), 2.93-2.86 (m, 1H), 2.78 (d, *J*=4.8 Hz, 3H), 2.61-2.56 (m, 4H), 2.10-2.04 (m, 1H), 1.96-1.91 ( m, 1H), 1.90-1.85 (m, 2H), 1.81-1.73 (m, 1H), 1.64-1.62 (m, 1H). | 41a, 1a |
| 82 | 467.2 | 8.43-8.40 (m, 1H), 7.79 (d, J=8.3 Hz, 1H), 7.59 (s, 1H), 7.48 (d, J=8.3 Hz, 1H), 7.26 (s, 1H), 5.73 (s, 1H), 4.66-4.50 (m, 2H), 3.89-3.79 (m, 2H), 3.30 (s, 2H), 2.94-2.92 (m, 4H), 2.80 (d, *J*=4.9 Hz, 3H), 2.56-2.54 (m, 4H), 2.48 (s, 3H). | 31a, 3a |

### Synthesis of compounds 4-1 and 4-2

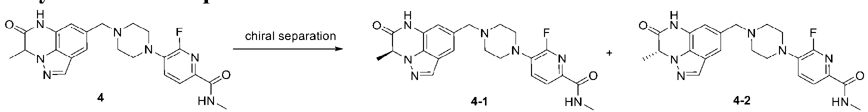

Compound **4** (45 mg) was separated by chiral column, column model DAICELCHIRALPAK^{®}IH, 250×25mm 10 µm, mobile phase A:B (10:1, A: ethanol (+0.2% 7.0mol/l ammonia methanol); B: acetonitrile (+0.2% 7.0mol/L ammonia methanol) ), to obtain compound **4-1** (15.98 mg) and compound **4-2** (17.69 mg);
Compound **4-1:** Peak time 3.662 min; ee% > 99. LCMS (ESI): m/z 438.3 [M+H]⁺; RT=0.467 min (6.00 min). ¹H-NMR (400 MHz, DMSO-d₆): δ 11.01 (s, 1H), 8.41-8.37 (m, 1H), 8.02 (s, 1H), 7.83 (d, J=7.2 Hz, 1H), 7.58-7.53 (m, 1H), 7.19 (s, 1H), 6.72 (s, 1H), 5.34-5.31 (m, 1H), 3.55 (s, 2H), 3.15 (s, 4H), 2.75 (d, J=3.6 Hz, 3H), 2.67 (d, J=2.8 Hz, 4H), 1.75 (dd, J=2.8 Hz, 6.4 Hz, 3H).

Compound **4-2:** Peak time 7.369 min; ee% > 94. LCMS(ESI): m/z 438.3 [M+H]⁺; RT=1.770 min (6.00 min). ¹H-NMR (400 MHz, DMSO-d₆): δ 11.04 (s, 1H), 8.40-8.38 (m, 1H), 8.03 (s, 1H), 7.84 (d, J=8.0 Hz, 1H), 7.55 (dd, J=8.0 Hz, 10.4 Hz, 1H), 7.19 (s, 1H), 6.73 (s, 1H), 5.36-5.31 (m, 1H), 3.56 (s, 2H), 3.15 (s, 4H), 2.76 (d, J=4.8 Hz, 3H), 2.54 (s, 4H), 1.75 (d, J=6.8 Hz, 3H).

Compounds 5-1/5-2, 6-1/6-2, 7-1/7-2, 11-1/11-2, 43-1/43-2, 45-1/45-2, 47-1/47-2, and 49-1/49-2 were obtained by chiral separation of compounds 5, 6, 7, 11, 43, 45, 47, and 49, respectively. Column model: DAICELCHIRALPAK^{®}IH, 250×25mm 10 µm, mobile phase: methanol (+0.1% 7.0mol/L ammonia methanol), ee%> 99. See Table 3 below. Among them, compounds 43, 45, 47, and 49 were prepared according to the synthesis method of compound 1, by replacing intermediates 10a and 2a with 18a and 1a (compound 43), 18a and 3a (compound 45), 19a and 1a (compound 47), and 19a and 3a (compound 49), respectively, and the resulting crude products were directly subjected to the above-mentioned chiral separation.

**Table 3**

| Com pd | LCMS (ESI): m/z [M+H]⁺ | ¹H NMR (400 MHz, DMSO-*d₆*) δ | Peak time (min) |
|---|---|---|---|
| 5-1 | 452.3 | 11.13 (s, 1H), 8.40-8.38 (m, 1H), 8.06 (s, 1H), 7.84 (d, J=7.6 Hz, 1H), 7.58-7.53 (m, 1H), 7.20 (s, 1H), 6.73 (s, 1H), 5.41-5.39 (m, 1H), 3.26 (s, 2H), 3.16 (s, 4H), 2.77 (s, 3H), 2.54 (d, J=4.0 Hz, 4H), 2.28-2.23 (m, 2H), 0.60 (t, J=7.2 Hz, 3H). | 3.07 |
| 5-2 | 452.3 | 11.53-11.44 (m, 1H), 8.42 (s, 1H), 8.23-8.03 (m, 1H), 7.86 (s, 1H), 7.72-7.21 (m, 1H), 6.82-6.71 (m, 1H), 5.46-5.38 (m, 1H), 3.73-3.62 (m, 2H), 3.16 (s, 4H), 2.77 (d, J=4.8 Hz, 3H), 2.58 (s, 4H), 2.28 (s, 2H), 0.61 (t, J=7.2 Hz, 3H). | 4.36 |
| 6-1 | 454.3 | 11.04 (s, 1H), 8.43-8.40 (m, 1H), 8.03 (s, 1H), 7.93 (d, *J*=8.4 Hz, 1H), 7.65 (d, *J*=8.0 Hz, 1H), 7.20 (s, 1H), 6.73 (s, 1H), 5.36-5.31 (m, 1H), 3.57 (s, 2H), 3.10 (s, 4H), 2.78 (d, *J*=4.4 Hz, 3H), 2.57 (s, 4H), 1.75 (d, *J*=7.2 Hz, 3H). | 3.99 |
| 6-2 | 454.3 | 11.04 (s, 1H), 8.41 (t, *J*=4.8 Hz, 1H), 8.03 (s, 1H), 7.93 (d, *J*=8.4 Hz, 1H), 7.65 (d, *J*=8.0 Hz, 1H), 7.20 (s, 1H), 6.73 (s, 1H), 5.36-5.31 (m, 1H), 3.57 (s, 2H), 3.10 (s, 4H), 2.79 (d, *J*=4.8 Hz, 3H), 2.57 (s, 4H), 1.75 (d, *J*=7.2 Hz, 3H). | 5.16 |
| 7-1 | 434.3 | 11.04 (s, 1H), 8.41 (d, *J*=4.8 Hz, 1H), 8.03 (s, 1H), 7.79 (d, *J*=8.0 Hz, 1H), 7.47 (d, *J*=8.4 Hz, 1H), 7.20 (s, 1H), 6.74 (s, 1H), 5.36-5.31 (m, 1H), 3.57 (s, 2H), 2.93 (s, 4H), 2.80 (d, *J*=4.8 Hz, 3H), 2.56 (s, 4H), 1.75 (d, *J=*6.8 Hz, 3H) | 2.75 |
| 7-2 | 434.3 | 11.04 (s, 1H), 8.40 (t, *J*=4.8 Hz, 1H), 8.03 (s, 1H), 7.79 (d, *J*=8.4 Hz, 1H), 7.47 (d, *J*=8.0 Hz, 1H), 7.20 (s, 1H), 6.74 (s, 1H), 5.36-5.31 (m, 1H), 3.58 (s, 2H), 2.93 (s, 4H), 2.80 (d, *J*=4.8 Hz, 3H), 2.57 (s, 4H), 1.75 (d, *J*=6.8 Hz, 3H). | 4.04 |
| 11-1 | 473.2 | 11.37 (s, 1H), 8.43-8.41 (m, 1H), 8.07 (s, 1H), 7.93 (d, *J=*8.0 Hz, 1H), 7.64 (d, *J*=8.4 Hz, 1H), 7.27 (d, *J*=5.2 Hz, 1H), 5.31 (q, *J=*6.8 Hz, 1H), 3.65 (s, 2H), 3.10-3.09 (m, 4H), 2.78 (d, *J*=5.2 Hz, 3H), 2.60-2.59 (m, 4H), 1.75 (d, *J*=6.8 Hz, 3H). | 3.78 |
| 11-2 | 473.2 | 11.37 (s, 1H), 8.43-8.41 (m, 1H), 8.08 (s, 1H), 7.93 (d, *J=*8.0 Hz, 1H), 7.64 (d, *J*=8.4 Hz, 1H), 7.27 (d, *J*=5.2 Hz, 1H), 5.31 (q, *J=*6.8 Hz, 1H), 3.65 (s, 2H), 3.10-3.09 (m, 4H), 2.78 (d, *J*=5.2 Hz, 3H), 2.60-2.59 (m, 4H), 1.75 (d, *J*=6.8 Hz, 3H). | 4.96 |
| 43-1 | 480.2 | 11.05 (s, 1H), 8.50-8.35 (m, 1H), 8.04 (s, 1H), 7.93 (d, iJ=8.1 Hz, 1H), 7.65 (d, *J*=8.2 Hz, 1H), 7.21 (s, 1H), 6.74 (s, 1H), 4.90 (d,*J*=7.4 Hz, 1H), 3.58 (s, 2H), 3.10 (s, 4H), 2.78 (d, *J*=4.8 Hz, 3H), 2.57 (s, 4H), 1.41-1.32 (m, 1H), 0.72 (dt, *J*=9.9, 5.0 Hz, 1H), 0.64-0.41 (m, 3H). | 4.44 |
| 43-2 | 480.2 | 11.05 (s, 1H), 8.43 (q, *J*=4.8 Hz, 1H), 8.04 (s, 1H), 7.93 (d, *J*=8.2 Hz, 1H), 7.65 (d, *J*=8.2 Hz, 1H), 7.21 (s, 1H), 6.74 (s, 1H), 4.90 (d, *J*=7.4 Hz, 1H), 3.58 (s, 2H), 3.10 (s, 4H), 2.78 (d, *J*=4.8 Hz, 3H), 2.57 (s, 4H), 1.41-1.32 (m, 1H), 0.73 (td, *J*=10.3, 4.9 Hz, 1H), 0.63-0.41 (m, 3H). | 5.24 |
| 45-1 | 460.2 | 11.05 (s, 1H), 8.41 (q, *J=*4.5 Hz, 1H), 8.04 (s, 1H), 7.79 (d, *J*=8.2 Hz, 1H), 7.47 (d, *J*=8.3 Hz, 1H), 7.21 (s, 1H), 6.75 (s, 1H), 4.90 (d, *J*=7.5 Hz, 1H), 3.58 (s, 2H), 2.93 (s, 4H), 2.79 (d, *J*=4.9 Hz, 3H), 2.57 (s, 4H), 2.48 (s, 3H), 1.42-1.32 (m, 1H), 0.73 (dq, *J*=10.2, 5.1 Hz, 1H), 0.52 (dddd, *J*=32.0, 20.2, 9.6, 5.9 Hz, 3H). | 3.26 |
| 45-2 | 460.2 | 11.05 (s, 1H), 8.41 (d, *J*=4.9 Hz, 1H), 8.04 (s, 1H), 7.79 (d, *J*=8.2 Hz, 1H), 7.47 (d, *J*=8.3 Hz, 1H), 7.21 (s, 1H), 6.75 (s, 1H), 4.90 (d, *J*=7.4 Hz, 1H), 3.58 (s, 2H), 2.93 (s, 4H), 2.80 (d, *J*=4.9 Hz, 3H), 2.57 (s, 4H), 2.48 (s, 3H), 1.36 (qd, *J*=8.0, 4.0 Hz, 1H), 0.73 (td, *J*=9.9, 4.7 Hz, 1H), 0.51 (dddd, *J*=20.0, 15.2, 10.5, 5.3 Hz, 3H). | 4.13 |
| 47-1 | 480.1 | 10.91 (s, 1H), 8.44 (d, *J*=4.8 Hz, 1H), 8.32 (s, 1H), 7.93 (d, *J*=8.2 Hz, 1H), 7.66 (d, *J*=8.2 Hz, 1H), 7.18 (s, 1H), 6.68 (s, 1H), 4.67 (d, *J*=9.0 Hz, 1H), 3.60 (s, 2H), 3.10 (s, 4H), 2.78 (d, *J*=4.8 Hz, 3H), 2.57 (s, 4H), 1.48-1.30 (m, 1H), 0.81-0.65 (m, 3H), 0.63-0.49 (m, 1H). | 3.69 |
| 47-2 | 480.1 | 10.91 (s, 1H), 8.44 (d, *J*=4.8 Hz, 1H), 8.32 (s, 1H), 7.93 (d, *J*=8.2 Hz, 1H), 7.66 (d, *J*=8.2 Hz, 1H), 7.18 (s, 1H), 6.68 (s, 1H), 4.67 (d, *J*=9.0 Hz, 1H), 3.60 (s, 2H), 3.10 (s, 4H), 2.78 (d, *J*=4.8 Hz, 3H), 2.57 (s, 4H), 1.48-1.31 (m, 1H), 0.80-0.64 (m, 3H), 0.63-0.49 (m, 1H). | 4.55 |
| 49-1 | 460.2 | 10.90 (s, 1H), 8.41 (d, *J*=4.8 Hz, 1H), 8.32 (s, 1H), 7.79 (d, *J*=8.2 Hz, 1H), 7.47 (d, *J*=8.2 Hz, 1H), 7.18 (s, 1H), 6.69 (s, 1H), 4.67 (d, *J*=9.0 Hz, 1H), 3.60 (s, 2H), 2.93 (s, 4H), 2.80 (d, *J*=4.8 Hz, 3H), 2.57 (s, 4H), 2.49 (s, 3H), 1.50-1.23 (m, 1H), 0.90-0.37 (m, 4H). | 2.99 |
| 49-2 | 460.2 | 10.91 (s, 1H), 8.41 (d, *J*=4.8 Hz, 1H), 8.31 (s, 1H), 7.79 (d, *J*=8.2 Hz, 1H), 7.47 (d, *J*=8.2 Hz, 1H), 7.19 (s, 1H), 6.69 (s, 1H), 4.67 (d, *J*=9.0 Hz, 1H), 3.60 (s, 2H), 2.93 (s, 4H), 2.80 (d, *J*=4.8 Hz, 3H), 2.57 (s, 4H), 2.49 (s, 3H), 1.50-1.23 (m, 1H), 0.89-0.37 (m, 4H). | 3.35 |

### Comparative Example

### Synthesis of Compound B1

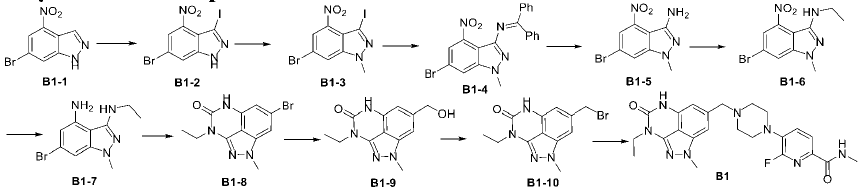

### Step 1: synthesis of 6-bromo-3-iodo-4-nitro-1H-indazole

B1-1 (3.0 g, 12.4 mmol) and N-iodosuccinimide (3.08 g, 13.7 mmol) in DMF (40 mL), was reacted at 60°C for 2 h. The reaction solution was added water and extracted with EA. EA phase was washed with saturated sodium chloride solution, concentrated and column chromatographed (PE/EA=3/1) to obtain B1-2 (3.9 g, yellow solid), yield: 85.4%, LCMS (ESI): m/z 367.7 [M-H]⁻; RT= 1.384 min (2.50 min).

### Step 2: synthesis of 6-bromo-3-iodo-1-methyl-4-nitro-1H-indazole

**B1-2** (4.0 g, 10.9 mmol) in DMF (25 mL) was added sodium hydride (1.1 g, 27.2 mmol) and iodomethane (2.3 g, 16.3 mmol). The mixture was stirred at rt for 2 h, quenched with saturated aqueous ammonium chloride solution, then extract with EA. The EA phase was washed with saturated sodium chloride solution, dried and concentrated. The residue was purified by column chromatography (PE/EA=5/1) to obtain **B1-3** (2.8 g, yellow solid), yield: 67.5%, LCMS (ESI): m/z 383.8 [M+H]⁺; RT= 1.593 min (2.50 min). ¹H NMR (400 MHz, DMSO-d₆): δ 8.53 (d, J=1.2 Hz, 1H), 8.02 (d, J=1.2 Hz, 1H), 4.14 (s, 3H).

### Step 3: synthesis of N-(6-bromo-1-methyl-4-nitro-1H-indazol-3-yl)-1,1-diphenylmethane imine

**B1-3** (2.0 g, 5.25 mmol), benzophenone imine (855 mg, 4.72 mmol), cesium carbonate (5.1 g, 15.7 mmol), 4,5-bis(diphenylphosphino)-9,9-dimethylxanthene (152 mg, 0.26 mmol) and tris(dibenzylideneacetone)dipalladium (240 mg, 0.26 mmol) were added to a 100 mL single-necked bottle containing 30 mL toluene. The mixture was stirred at 80°C overnight, filtered and concentrated. Th residue was purified by column chromatograph (PE/EA=10/1) to obtain **B1-4** (1.3 g, yellow solid). Yield: 57.1 %, LCMS (ESI): m/z 435.0 [M+H]⁺; RT= 1.534 min (2.50 min).

### Step 4: synthesis of 6-bromo-1-methyl-4-nitro-1H-indazole-3-amine

The solution of **B1-4** (1.1 g, 2.5 mmol) in 8 mL of HCl/EA was stirred at rt for 2 h, filtered, washed with EA, and dried to obtain **B1-5** (600 mg, yellow solid), yield: 87.7%, LCMS (ESI): m/z 272.9 [M+H]⁺; RT= 1.180 min (2.50 min).

### Step 5: synthesis of 6-bromo-N-ethyl-1-methyl-4-nitro-1H-indazole-3-amine

The mixture of **B1-5** (500 mg, 1.93 mmol), acetaldehyde (127 mg, 2.89 mmol) and acetic acid (two drops) was stirred at 60°C for 1h, then add sodium cyanoborohydride (363 mg, 5.78 mmol). The mixture was added water, extracted with EA, washed with saturated sodium chloride solution, dried and concentrated. The residue was purified by column chromatography (PE/EA=3/1) to obtain **B1-6** (500 mg, yellow solid), yield: 87.1%, LCMS (ESI): m/z 301.0 [M+H]⁺; RT= 1.690 min (2.50 min).

### Step 6: synthesis of 6-bromo-N3-ethyl-1-methyl-1H-indazole-3,4-diamine

**B1-6** (670 mg, 2.25 mmol) and ammonium chloride (962 mg, 18.0 mmol) were added to a 100 mL single-mouth bottle containing 10 mL methanol and 1 mL water, and zinc powder (1.18 g, 18.0 mmol) was added under ice bath. The mixture was reacted at rt for 2h, filtered. The filtrate was diluted with water, extracted with EA. The organic phase was washed with water and saturated sodium chloride solution, dried with anhydrous sodium sulfate, and concentrated to obtain **B1-7** (840 mg, yellow solid), LCMS (ESI): m/z 271.0 [M+H]⁺; RT= 1.179 min (2.50 min).

### Step 7: synthesis of 7-bromo-3-ethyl-1-methyl-1,5-dihydropyrazolo[3,4,5-d]quinazolin-4(3H)-one

**B1-7** (560 mg, 2.09 mmol) and carbonyldiimidazole (1.69 g, 10.4 mmol) were added to a 100 mL single-mouth bottle containing 15 mL N-methylpyrrolidone. The mixture was heated at 140°C for 2h, diluted with water, precipitated, filtered and dried to obtain **B1-8** (380 mg, yellow solid), yield: 61.9%. LCMS (ESI): m/z 297.1 [M+H]⁺; RT=0.890 min (2.50 min). 1H NMR (400 MHz, DMSO-d6): δ 10.51 (s, 1H), 7.06 (s, 1H), 6.16 (s, 1H), 3.82- 3.77 (m, 5H), 1.24-1.20 (m, 3H).

### Step 8: synthesis of 3-ethyl-7-(hydroxymethyl)-1-methyl-1,5-dihydropyrazolo[3,4,5-d]quinazolin-4(3H)-one

**B1-8** (200 mg, 0.68 mmol), tributyltin methanol (262 mg, 0.82 mmol) and XPhos Pd G2 (27 mg, 0.034 mmol) in dioxane (10 mL) was reacted at 80°C for 16 h. The reaction solution was concentrated and purified by column chromatography (PE/EA=1/1) to obtain **B1-9** (140 mg, yellow solid). Yield: 83.6%, LCMS (ESI): m/z 247.2 [M+H]⁺; RT= 0.561 min (2.50 min).

### Step 9: synthesis of 7-(bromomethyl)-3-ethyl-1-methyl-1,5-dihydropyrazolo[3,4,5-d]quinazolin-4(3H)-one

The mixture of **B1-9** (70 mg, 0.28 mmol), triphenylphosphine (223 mg, 0.85 mmol) and carbon tetrabromide (189 mg, 0.57 mmol) in 8 mL DCM was reacted at rt for 2 h, then concentrated to obtain **B1-10** (70 mg, yellow solid). LCMS (ESI): m/z 3B1.0 [M+H]⁺; RT= 1.438 min (2.50 min).

### Step 10: synthesis of 5-(4-((3-ethyl-1-methyl-4-oxo-1,3,4,5-tetrahydropyrazolo[3,4,5-d]quinazolin-7-yl)methyl)piperazine-1-yl)-6-fluoro-N-methylpicolinamide

**B1-10** (80 mg, 0.26 mmol), intermediate **2a** (71 mg, 0.26 mmol) and DIEA (166 mg, 1.29 mmol) were added to a 50 mL single-necked bottle containing 5 mL of acetonitrile. The mixture was reacted at 70°C for 2 h. The reaction solution was purified by Pre-HPLC (formic acid) to obtain **B1** (37.72 mg, white solid). The yield was 31.4%. LCMS(ESI): m/z 467.4 [M+H]⁺; RT=3.215 min (6.00 min).¹H NMR (400 MHz, DMSO-d6): δ 10.31 (s, 1H), 8.40 (d, J=4.8 Hz, 1H), 7.86-7.83 (m, 1H), 7.59-7.54 (m, 1H), 6.70 (s, 1H), 6.13 (s, 1H), 3.82-3.77 (m, 5H), 3.51 (s, 2H), 3.17 (s, 4H), 2.77 (d, J=4.8 Hz, 3H), 2.55 (s, 4H), 1.24-1.20 (m, 3H).

### Synthesis of Compound B2

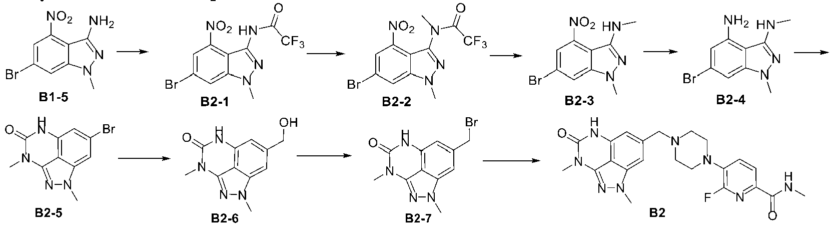

### Step 1: synthesis of N-(6-bromo-1-methyl-4-nitro-1H-indazol-3-yl)-2,2,2-trifluoroacetamide

The mixture of **B1-5** (1.8 g, 6.67 mmol), DIEA (2.58 g, 20.0 mmol) in DCM (20 mL) was added trifluoroacetic anhydride (2.10 g, 10.0 mmol). The mixture was stirred at rt for 2 h, added water and extract with EA. The EA phase was washed with saturated sodium chloride solution, dried and concentrated to obtain **B2-1** (2.3 g, yellow solid). LCMS (ESI): m/z 368.9 [M+H]⁺; RT= 1.352 min (2.50 min).

### Step 2: synthesis of N-(6-bromo-1-methyl-4-nitro-1H-indazol-3-yl)-2,2,2-trifluoro-N-methylacetamide

**B2-1** (2.3 g, 6.28 mmol), cesium carbonate (4.08 g, 12.6 mmol) and iodomethane (1.34 g, 9.42 mmol) in DMF (25 mL) was reacted at 30°C for 2 h. Water was added to the reaction solution, which was extracted with EA, washed with saturated sodium chloride solution, dried and concentrated to obtain **B2-2** (2.3 g, yellow solid). LCMS (ESI): m/z 380.9 [M+H]⁺; RT= 1.519 min (2.50 min).

### Step 3: synthesis of 6-bromo-N,1-dimethyl-4-nitro-1H-indazole-3-amine

B2-2 (2.6 g, 6.8 mmol) and sodium hydroxide aqueous solution (2 mL, 6N) in 30 mL of methanol was stirred at rt for 30 minutes. The reaction solution was filtered, and the filter cake was washed with water and methanol respectively. After drying, the product **B2-3** (1.5 g, black solid) was obtained. Yield:77.3%. LCMS (ESI): m/z 287.0 [M+H]⁺; RT= 1.495 min (2.50 min). 1H NMR (400 MHz, DMSO-d6): δ 8.25 (s, 1H), 7.83 (s, 1H), 5.58-5.54 (m, 1H), 3.88 (s, 3H), 2.87 (d, J=5.2 Hz, 3H).

### Step 4: synthesis of 6-bromo-N,1-dimethyl-1H-indazole-3,4-diamine

**B2-3** (1.4 g, 4.93 mmol) and ammonium chloride (2.B1 g, 39.4 mmol) were added to a 100 mL single-mouth bottle containing methanol (30 mL) and water (3 mL). Zinc powder (2.58 g, 39.4 mmol) was added at 0°C. The mixture was reacted at rt for 2 h, filtered. The filtrate was concentrated to dryness, diluted with water, extracted with EA, and the organic phase was washed with water and saturated brine, dried over anhydrous sodium sulfate, and concentrated to obtain **B2-4** (840 mg, brown solid). LCMS (ESI): m/z 255.0 [M+H]⁺; RT= 0.558 min (2.50 min).

### Step 5: synthesis of 7-bromo-1,3-dimethyl-1,5-dihydropyrazolo[3,4,5-d]quinazolin-4(3H)-one

**B2-4** (800 mg, 3.15 mmol) and carbonyldiimidazole (2.55 g, 1.57 mmol) were added to a 50 mL single-necked bottle containing 15 mL N-methylpyrrolidone. The mixture was reacted at 140°C for 2 h, diluted with water. The precipitate was filtered and dried to obtain **B2-5** (700 mg, gray solid). The yield was 79.3%. LCMS (ESI): m/z 281.0 [M+H]⁺; RT=1.098 min (2.50 min). 1H NMR (400 MHz, DMSO-d6): δ 10.52 (s, 1H), 7.05 (s, 1H), 6.16 (s, 1H), 3.77 (s, 3H), 3.22 (s, 3H).

### Step 6: synthesis of 7-(hydroxymethyl)-1,3-dimethyl-1,5-dihydropyrazolo[3,4,5-d]quinazolin-4(3H)-one

**B2-5** (300 mg, 1.07 mmol), tributyltinmethanol (413 mg, 1.29 mmol) and XPhos Pd G2 (42 mg, 0.05 mmol) in dioxane (10 mL) was stirred at 80°C for 16 h. The reaction solution was concentrated. The residue was purified by column chromatography (PE/EA=1/1) to obtain **B2-6** (220 mg, black solid). Yield: 88.5%, LCMS (ESI): m/z 233.1 [M+H]⁺; RT= 0.924 min (2.50 min).

### Step 7: synthesis of 7-(bromomethyl)-1,3-dimethyl-1,5-dihydropyrazolo[3,4,5-d]quinazolin-4(3H)-one

**B2-6** (35 mg, 0.15 mmol), triphenylphosphine (B19 mg, 0.45 mmol) and carbon tetrabromide (100 mg, 0.30 mmol) in DCM (8 mL) was stirred at rt for 2 h. The reaction solution was concentrated to obtain **B2-7** (35 mg, yellow solid). LCMS (ESI): m/z 297.0 [M+H]⁺; RT= 1.039 min (2.50 min).

### Step 8: synthesis of 5-(4-((1,3-dimethyl-4-oxo-1,3,4,5-tetrahydropyrazolo[3,4,5-d]quinazolin-7-yl)methyl)piperazine-1-yl)-6-fluoro-N-methylpicolinamide

**B2-7** (35 mg, 0.24 mmol), intermediate **2a** (35 mg, 0.24 mmol) and DIEA (77 mg, 1.19 mmol) in 5 mL of acetonitrile was stirred at 70°C for 2 h. The reaction solution was purified by Pre-HPLC (formic acid) to obtain **B2** (14.35 mg, white solid), with a yield of 27.7%. LCMS(ESI): m/z 453.4 [M+H]⁺; RT=3.166 min (6.00 min).¹H NMR (400 MHz, DMSO-d₆): δ 10.35 (s, 1H), 8.41-8.38 (m, 1H), 7.85-7.83 (m, 1H), 7.58-7.54 (m, 1H), 6.70 (s, 1H), 6.13 (s, 1H), 3.76 (s, 3H), 3.51 (s, 2H), 3.24 (s, 3H), 3.17 (s, 4H), 2.76 (d, J=4.8 Hz, 3H), 2.54 (s, 4H).

### Synthesis of Compound B3

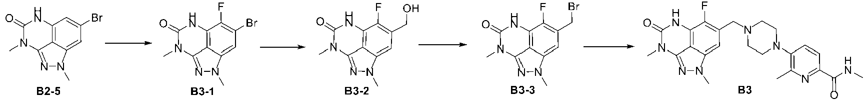

### Step 1: synthesis of 7-bromo-6-fluoro-1,3-dimethyl-1,5-dihydropyrazolo[3,4,5-de]quinazolin-4(3H)-one

The mixture of **B2-5** (200 mg, 0.71 mmol) and N-fluorobisbenzenesulfonamide (293 mg, 0.93 mmol) in acetic acid (10 mL) was reacted at 100°C for 16 h. After concentration, **B3-1** (50 mg, red-brown solid) was prepared using Pre-HPLC (NH₄HCO₃). Yield:23.5%. LCMS (ESI): m/z 299.0 [M+H]⁺; RT= 1.403 min (2.50 min). ¹H NMR (400 MHz, DMSO-d₆): δ 7.19 (d, J=3.6 Hz, 1H), 3.77 (s, 3H), 3.22 (s, 3H).

### Step 2: synthesis of 6-Fluoro-7-(hydroxymethyl)-1,3-dimethyl-1,5-dihydropyrazolo[3,4,5-de]quinazolin-4(3H)-one

**B3-1** (10 mg, 0.03 mmol), tributyltin methanol (13 mg, 0.04 mmol) and XPhos Pd G2 (11 mg, 0.014 mmol) in dioxane (5 mL) was reacted at 80°C for 16 h. The reaction solution was concentrated and slurried with pure EA to obtain **B3-2** (6 mg, brown solid). Yield: 71.5%, LCMS (ESI): m/z 251.1 [M+H]⁺; RT= 0.896 min (2.50 min).

### Step 3: synthesis of 7-(Bromomethyl)-6-fluoro-1,3-dimethyl-1,5-dihydropyrazolo[3,4,5-de]quinazolin-4(3H)-one

**B3-2** (11 mg, 0.044 mmol), triphenylphosphine (35 mg, 0.13 mmol) and carbon tetrabromide (29 mg, 0.088 mmol) in DCM (8 mL) was reacted at rt for 2 h. The reaction solution was concentrated to obtain **B3-3** (11 mg, yellow solid). Crude product, LCMS (ESI): m/z 313.0 [M+H]⁺; RT= 1.370 min (2.50 min).

### Step 4: synthesis of 5-(4-(((6-fluoro-1,3-dimethyl-4-oxo-1,3,4,5-tetrahydropyrazolo[3,4,5-de]quinazolin-7-yl)methyl)piperazin-1-yl)-N,6-dimethylpicolinamide

**B3-3** (30 mg, 0.1 mmol), intermediate **3a** (47 mg, 0.15 mmol) and DIEA (77 mg, 0.6 mmol) in CAN (5 mL) was reacted at 70°C for 1.5 h. The mixture was purified by Pre-HPLC (formic acid) to obtain **B3** (32 mg, white solid), with a yield of 71.3%. LCMS (ESI): m/z 467.8 [M+H]⁺; RT=3.362 min (6.00 min).¹H NMR (400 MHz, DMSO-d₆): δ 10.68 (s, 1H), 8.55-8.51 (m, 1H), 7.76 (d, J=8.4 Hz, 1H), 7.45 (d, J=8.4 Hz, 1H), 6.75 (d, J=3.2 Hz, 1H), 3.73 (s, 3H), 3.61 (s, 2H), 3.20 (s, 3H), 2.92 (s, 4H), 2.78 (d, J=4.8 Hz, 3H), 2.61 (s, 4H), 2.44 (s, 3H).

### Biological Examples of Implementation

### Experimental Example 1: Assessment of PARP1/2 inhibitory activity

The PARP1/2 inhibitory activity of the compounds of the present disclosure was tested in an assay using Histone as a substrate.

Aim of the assay: to test the IC₅₀ values of the compounds of the present application for inhibition of PARP1/2 enzymatic activity according to established experimental methods. AZD-2281 (Olaparib) was used as a positive control.

### Experimental Reagents:

Recombinant human PARP1 protein (Abcam, cat. ab279663); recombinant human PARP2 protein (BPS, cat. 80502); recombinant histone H1 (Active Motif, cat. 81126); NAD+, Biotin-Labelled (BPS, cat. 80610); SuperBlock (TBS) Blocking Buffer (Thermo Scientific^{™}, cat. 37535); Streptavidin (HRP) (Abcam, cat. ab7403); LumiGLO^{®} Peroxidase Chemiluminescent Substrate Kit (Seracare, cat. 5430-0040); 20×PBS (CST, cat. 9808S); 20×PBST (CST, cat. 9809S); AZD2281(Selleck, cat. S1060)

### Experimental method I: PARP1 inhibitory activity

1. Compound Configuration: Diluted the compounds with DMSO in 384-well plates to a 1000-fold final concentration and set aside.
2. Inclusion microtiter plate:
   1) Diluted Histone with PBS, added 25uL of Histone mixture to each well and incubated for 2 h. 2) Washed each well 5 times using PBST solution. Removed the solution on a clean paper towel.3) Added 75 uL of Blocking buffer to each well and incubated for 1 h at rt. 4) Washed each well 5 times using PBST solution. Removed the solution on a clean paper towel.
3. Ribosylation reaction:
   1) Transfered 25 nL of 1000× final concentration of compound to a 384 reaction plate; added 25 nL of 100% DMSO to Min control wells and Max control wells. 2) 1× Assay buffer to prepare 2.5× final concentration of PARP1 solution. 3) Added 10 uL of enzyme solution to the compound wells and Max control wells; added 10 uL of 1× Assay buffer. 4) Centrifuged at 1000 rpm for 60 s and incubated at rt for 15 min. 5) Prepared 1.67× final concentration of substrate solution with 1× Assay buffer, 500 µM NAD+ was added to the substrate solution, and 15 µL of substrate solution was added to each well to start the reaction. 6) Centrifuged at 1000 rpm for 60 s and incubated at rt for 2 h. 7) Each well was washed 5 times with PBST solution. Removed the solution on a clean paper towel.
4. Detection:
   1) Configured Streptavidin-HRP solution, added 25 µL to each well, centrifuged at 1000 rpm for 60 sec, and incubated at rt for 30 min. 2) Washed each well 5 times using PBST solution. Removed the solution on a clean paper towel. 3) Added 50 µL of ELISA Chemiluminescent Substrate per well. 4) Centrifuged at 1000 rpm for 60 seconds and read with EnSight after 5 min.
5. Data analysis:

The inhibition rate was calculated using the following formula: % inhibition = (max signal - compound signal) / (max signal - min signal) × 100, where 'min signal' is the mean value of negative control wells and 'max signal' is the mean value of positive control wells.

Fitting the quantitative effect curve: using the log value of the concentration as the X-axis and the percentage inhibition as the Y-axis, the log(inhibitor) vs. response-variable slope of the analytical software GraphPadPrism5 was used to fit the quantitative effect curve to arrive at the IC₅₀ value for the inhibition of the enzyme activity by the compounds of the present disclosure. The fitting formula is: Y=Bottom+ (Top-Bottom)/(1+10^((logIC₅₀-X)*HillSlope)). Among them, "Top" and "Bottom" are the maximum inhibition rate and the minimum inhibition rate respectively; "HillSlope" is the absolute value of the maximum slope of the curve.

### Experimental method II: PARP2 inhibitory activity

The PARP2 inhibitory activity assay was performed as in Experimental Method 1, except that recombinant human PARP2 solution was used instead of PARP1 solution used in step 2 '2)' of '3. Glycosylation reaction', and 500 µM NAD+ was not added in step 5 of the glycosylation reaction.

**Table 4. IC₅₀ of the inhibitory effect of the compounds of the present invention on PARP1/2 enzyme**

| Compd | PARP1 IC₅₀ (nM) | PARP2 IC₅₀ (nM) | PARP1/2 Fold selectivity | Compd | PARP1 IC₅₀ (nM) | PARP2 IC₅₀ (nM) | PARP1/2 Fold selectivity |
|---|---|---|---|---|---|---|---|
| 5 | 0.78 | 215.4 | 276 | 43-1 | 1.67 | 574.9 | 344.5 |
| 5-1 | 1.35 | 347.4 | 257.5 | 43-2 | 1.04 | 139.1 | 133.3 |
| 5-2 | 1.03 | 206.8 | 201 | 45-1 | 1.65 | 719.6 | 435 |
| 6 | 0.42 | 300.8 | 716.2 | 45-2 | 1.5 | 222.5 | 148 |
| 6-1 | 0.42 | 437.9 | 1047 | 47-1 | 0.68 | 901.3 | 1326 |
| 6-2 | 0.27 | 218.7 | 820 | 47-2 | 1.21 | 281.5 | 232.5 |
| 7 | 0.3 | 327.2 | 1090.7 | 49-1 | 0.71 | 1310 | 1852 |
| 7-1 | 0.48 | 499.3 | 1043 | 49-2 | 1.53 | 431 | 281.5 |
| 7-2 | 0.32 | 325.7 | 1004 | 51 | 0.88 | 2646 | 3023 |
| 8 | 0.67 | 673.7 | 1005.5 | 52 | 1.27 | 347.5 | 273.8 |
| 9 | 0.79 | 855.8 | 1083.3 | 53 | 2.16 | 45.5 | 21.1 |
| 11 | 0.18 | 845.9 | 4699 | 54 | 0.4 | 45.2 | 21.1 |
| 11-1 | 0.39 | 1590 | 4050 | 55 | 2.64 | 1009 | 382 |
| 11-2 | 0.42 | 618.6 | 1463 | 56 | 3.34 | 1218 | 364.5 |
| 12 | 0.41 | 1355 | 3316 | 57 | 1.31 | 265.7 | 202.8 |
| 16 | 1.2 | 2404 | 2046 | 58 | 0.99 | 204 | 206 |
| 17 | 0.71 | 83.7 | 117.9 | 59 | 2.8 | 137 | 49.8 |
| 19 | 1.42 | 731.7 | 516 | 60 | 1.89 | 1207 | 638.6 |
| 26 | 0.28 | 1442 | 5150 | 61 | 2.2 | 518.6 | 237 |
| 27 | 1.20 | 1763 | 1469 | 62 | 3.3 | 1405 | 422.3 |
| 30 | 1.24 | 210.9 | 170.8 | 63 | 2.1 | 1826 | 827.4 |
| 35 | 0.39 | 200.9 | 509.9 | 65 | 15.6 | 2598 | 167 |
| 37 | 0.24 | 8.7 | 36.3 | 66 | 3.3 | 1378 | 417.5 |
| 38 | 0.25 | 28.7 | 114.8 | 72 | 4.9 | 474.2 | 97.6 |
| 39 | 0.21 | 9.7 | 46.2 | 73 | 2.9 | 205.9 | 70 |
| 40 | 0.23 | 81 | 353.6 | 80 | 3.9 | 413.6 | 104.8 |
| 41 | 0.1 | 71.9 | 719 | 81 | 4.2 | 2405 | 574.1 |
| 42 | 0.86 | 265.8 | 309 | | | | |
| B1 | 1.4 | 3.7 | 2.64 | B2 | 0.3 | 9.7 | 32.3 |
| B3 | 0.82 | 61.9 | 75.5 | AZD-2281 | 26.85 | 0.35 | 0.013 |

| | | | | | | | |
|---|---|---|---|---|---|---|---|
| Note: "/" indicates not detected. | | | | | | | |

As shown in Table 4, the compounds of the present invention have high selectivity for PARP1, good selectivity for PARP1, and a selectivity multiple of ≥100, which can reduce the toxicity caused by PARP2 without significantly reducing the efficacy. In addition, the high selectivity of the compounds of the present invention for PARP1 is significantly better than that of the comparative compounds B1, B2, B3 and AZD-2281.

### Example 2: MDA-MB-436 cell proliferation inhibition test

Human breast cancer MDA-MB-436 (purchased from ATCC) cells were cultured in DMEM medium (supplemented with 10% fetal bovine serum with 1% dual antibody) at 37°C, 5% CO₂. The cells were taken from the logarithmic growth phase, digested and configured with a certain concentration of cell suspension, which was inoculated into 96-well plates, 100 µL of cell suspension was added to each well of the 96-well plates, incubated overnight, then different concentrations of compounds were added, and the cells were incubated in the cell culture incubator for 7 days. At the end of incubation, 50 µL of CellTiter-Glo reagent was added to each well according to CellTiter-Glo reagent, mixed with a microplate shaker for 2 min, and left at rt for 60 min, then the fluorescence value was read with a Spark^{®} multimode microplate reader, and the fluorescence value was calculated according to the formula: [(1-(RLUcompound-RLUblank)/(RLUcontrol-RLUblank)) × 100%] to calculate the cell proliferation inhibition rate. IC₅₀ values were obtained by fitting using GraphPad Prism 6.0 software.

**Table 5. MDA-MB-436 cell proliferation inhibitory activity of the compounds of the present invention**

| Compd | MDA-MB-436 IC₅₀ (nM) | Compd | MDA-MB-436 IC₅₀ (nM) | Compd | MDA-MB-436 IC₅₀ (nM) |
|---|---|---|---|---|---|
| 5 | 15.4 | 5-1 | 27.4 | 5-2 | 14.8 |
| 6 | 9.0 | 6-1 | 5.5 | 6-2 | 3.2 |
| 7 | 6.7 | 7-1 | 7.9 | 7-2 | 2.6 |
| 8 | 12.7 | 9 | 15.4 | 11 | 6.5 |
| 11-1 | 3.1 | 11-2 | 2.9 | 12 | 19.4 |
| 16 | 73.1 | 17 | 1.4 | 19 | <1.52 |
| 25 | 32.5 | 27 | 22.4 | 30 | 2.7 |
| 33 | <1.52 | 34 | 2.3 | 35 | 213.3 |
| 37 | <1.52 | 38 | <1.52 | 39 | <1.52 |
| 40 | 17.9 | 41 | 4.4 | 42 | 1.0 |
| 43-1 | 8.7 | 43-2 | 1.7 | 45-1 | 7.7 |
| 45-2 | 2.4 | 47-1 | 20.2 | 47-2 | 16.7 |
| 49-1 | 20.4 | 49-2 | 18.0 | 51 | 7.7 |
| 52 | 21.5 | 53 | 2.2 | 54 | <1.52 |
| 56 | 15 | 57 | 1.3 | 58 | 1.2 |
| 59 | 6.5 | 61 | 16.2 | 62 | 20.9 |
| 63 | 16.8 | 64 | 17.5 | 66 | 48.1 |
| 67 | 392.2 | 68 | 157.4 | 69 | 719.5 |
| 71 | 135.1 | 73 | 3.2 | 79 | 21.8 |
| 80 | 18.7 | 81 | 4.6 | | |

The experimental results show that the compounds of the present invention have significant proliferation inhibition activity on MDA-MB-436 cells.

### Experimental Example 3: Preliminary Pharmacokinetic Tests

1. Healthy ICR mice were taken and the tested compounds were administered intravenously (1 mg/kg) and by gavage (5 mg/kg). Nine male mice weighing 30-35 g for each route of administration were randomly divided into three groups of three mice each.
   The mice were fasted for 12h before the test and allowed to drink water freely. The mice were fed 4h after the administration.
2. Blood collection time point and sample processing
   Intravenous and gavage administration: 0.25h, 0.5h, 1.0h, 2.0h, 3.0h, 4.0h, 6.0h, 8.0h and 24h after administration.
   Blood was collected continuously, and 3 animals were collected at each time point. Plasma collection and processing: 30-40 µL of venous blood was collected from the mouse retroorbital venous plexus at the above set time points, placed in an EDTA-K2 test tube, centrifuged at 3500 rpm for 10 min, and plasma was separated and frozen in a -20°C refrigerator.
3. Sample testing and data analysis
   The concentration of the compounds in mouse plasma was determined by LC/MS/MS. Pharmacokinetic parameters after administration were calculated using the non-atrial model of Phoenix 8.3 software (Pharsight, USA).
4. Experimental results

**Table 6. Pharmacokinetic parameters of the compounds of the present invention in mouse plasma**

| Compound | Administration | T_{1/2} (hr) | CL (mL/min/kg) | AUC₀₋ₜ (ng·hr/mL) | Bioavailability (%) |
|---|---|---|---|---|---|
| 6 | i.v.(1mg/kg) | 1.3 | 3.3 | 5032 | 116.8 |
| | p.o.(5mg/kg) | 2.7 | / | 28712 | |
| 7 | i.v.(1mg/kg) | 3.4 | 0.9 | 17755 | 93.7 |
| | p.o.(5mg/kg) | 4.1 | / | 71223 | |
| 10 | i.v.(1mg/kg) | 3.4 | 2.1 | 7770 | 50.9 |
| | p.o.(5mg/kg) | 3.6 | / | 24136 | |
| 40 | i.v.(1mg/kg) | 3.6 | 3.8 | 4412 | 124.2 |
| | p.o.(5mg/kg) | 3.1 | / | 31105 | |

| | | | | | |
|---|---|---|---|---|---|
| Note: i.v.: intravenous injection; p.o.: oral administration. | | | | | |

The experimental results show that the pharmacokinetics of the compounds of the present invention in mice after oral administration show a longer half-life (T_{1/2}), lower clearance rate (CL) and higher bioavailability.

### Experimental Example 4: Test of blood-brain barrier properties in rats

The compounds of the present invention were prepared into an aqueous solution containing 5% DMSO and 95% (containing 20% hydroxypropyl-β-cyclodextrin), and intravenously injected into 3 Wistar Han rats (male) at 1 mg/kg. After 4 h, carotid artery blood samples were collected and brain tissues were collected after brain perfusion. The drug concentration was determined by LC-MS/MS method, and the results are shown in the following table:

**Table 7. Brain permeability of the compounds of the present invention in the blood-brain barrier of rats**

| Compound | concentration in brain tissue (ng/g) | concentration in plasma (ng/mL) | Ratio % (brain/blood) |
|---|---|---|---|
| 7 | 51 | 379 | 13.8 |

### Experimental Example 5: PDE3A enzyme activity test

Phosphodiesterase 3A (PDE3A) is mainly distributed in the heart, vascular smooth muscle and platelets, and has the functions of regulating myocardial contractility, vascular smooth muscle contraction, platelet aggregation, etc. If the compound has a strong inhibitory effect on PDE3A, it may cause side effects such as increased cardiac contractility, decreased blood pressure, arrhythmia, and thrombocytopenia (References: Bowes, J.; Brown, A. J.; Hamon, J.; Jarolimek, W.; Sridhar, A.; Waldron, G.; Whitebread, S. Reducing safety-related drug attrition: the use of in vitro pharmacological profiling. Nat Rev Drug Discov 2012, 11 (12), 909-922.)

Experimental method: Different concentrations of the compounds, PDE3A protein (ICE, G13020203013) (0.2 nM) and FAM-cAMP (BPS, 60200) (200 nM) were added to a 384-well plate (Corning, 4514) and reacted at rt for 60 minutes. Added 15 µL of binding reagent (IMAP FP IPP Explorer Kit, Molecular Devices, R8124) mixture to each well and incubated the reaction at rt for 60 minutes. Used Microplate Reader (BMG, PHERAstar FSX) to read the FP signal (ex 490 nm/em 520 nm).

**Table 8. Inhibitory effect of the compounds of the present invention on PDE3A**

| Compd | PDE3A IC₅₀ (nM) | Compd | PDE3A IC₅₀ (nM) | Compd | PDE3A IC₅₀ (nM) |
|---|---|---|---|---|---|
| 5-2 | >10000 | 6-2 | 238.7 | 7-1 | 1194 |
| 7-2 | 543.9 | 11-2 | 166.4 | 19 | 433.2 |
| 40 | 635.5 | 47-1 | >10000 | 47-2 | >10000 |
| 49-2 | >10000 | 54 | 427.5 | 56 | 878.1 |
| 59 | >10000 | 61 | 1438 | | |

The experimental results show that some compounds of the present invention do not have a strong inhibitory effect on PDE3A.

In summary, the compounds of the present invention have high selectivity for PARP1. The inventors also unexpectedly found that the compounds of the present invention show high brain permeability in the rat blood-brain barrier test (brain/blood ratio greater than 10%), and the compounds of the present invention have good physical and chemical stability, good bioavailability (such as low clearance rate) and good drugability. Therefore, the compounds of the present invention have fewer side effects than olaparib (AZD-2281) and have high clinical application value.

The above embodiments are only used to illustrate the technical scheme of the present invention, not to limit it. Although the present invention is described in detail with reference to the above embodiments, ordinary technicians in the field should understand that the technical schemes recorded in the above embodiments can be modified, or some or all of the technical features therein can be replaced by equivalents without departing from the spirit and essence defined by the claims of the present invention; and these modifications or replacements are still within the scope defined by the claims of the present invention.

## Claims

1. A compound of formula (I), or a stereoisomer, a geometric isomer, a tautomer, a pharmaceutically acceptable salt or a hydrate thereof, 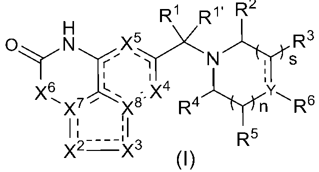 wherein,
-̅ -̅ -̅ -̅ -̅ -̅ is a single bond or a double bond;
X² is selected from N, -C(=O) and CR^{8a};
X³ is selected from N, O and CR^{8b};
X⁴ is selected from N and CR⁹;
X⁵ is selected from N and CR¹⁰;
X⁶ is selected from NR^{7c} and CR¹⁴R¹⁵.
X⁷ and X⁸ are each selected from C and N, provided that X⁷ and X⁸ are not C at the same time, X² and X³ are not N at the same time;
R^{7c} is selected from hydrogen, substituted or unsubstituted C₁-C₆ alkyl, substituted or unsubstituted C₃-C₈ cycloalkyl, substituted or unsubstituted C₁-C₆ alkoxy, substituted or unsubstituted 3 to 8-membered heterocyclyl, substituted or unsubstituted C₂-C₆ alkynyl; preferably, R^{7c} is selected from hydrogen, C₁-C₄ alkyl being unsubstituted or substituted with halogen, hydroxyl or C₃-C₆ cycloalkyl, unsubstituted or halogen substituted C₃-C₆ cycloalkyl, C₁-C₆ alkoxy, 3 to 6 membered heterocyclyl, C₂-C₆ alkynyl;
R^{8a} and R^{8b} are each independently selected from hydrogen, halogen, cyano, substituted or unsubstituted C₁-C₆ alkyl, substituted or unsubstituted C₃-C₆ cycloalkyl; preferably, R^{8a} and R^{8b} are each independently selected from hydrogen, fluorine, chlorine, methyl, ethyl and cyclopropyl;
R⁹ and R¹⁰ are each independently selected from hydrogen, halogen, cyano, substituted or unsubstituted C₁-C₆ alkyl, substituted or unsubstituted C₁-C₆ alkoxy; preferably, R⁹ and R¹⁰ are each independently selected from hydrogen, halogen, cyano and C₁-C₄ alkyl; more preferably, R⁹ and R¹⁰ are each independently selected from hydrogen, fluorine, chlorine and methyl;
R¹⁴ and R¹⁵ are each independently selected from hydrogen, substituted or unsubstituted C₁-C₆ alkyl, substituted or unsubstituted C₃-C₆ cycloalkyl; or R¹⁴ and R¹⁵ together with the carbon atoms bonded thereto form C₃-C₆ cycloalkyl;
R¹, R^{1'}, R² and R³ are each independently selected from hydrogen, substituted or unsubstituted C₁-C₆ alkyl;
R⁴ and R⁵ are each independently selected from hydrogen, substituted or unsubstituted C₁-C₆ alkyl; or R⁴ and R⁵ together with the carbon atoms bonded thereto form C₃-C₆ cycloalkyl;
s, n are each independently selected from 0, 1 and 2;
Y is N, C or CH;
R⁶ is selected from: 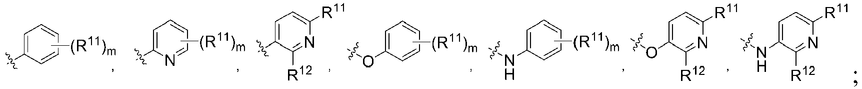
R¹¹ is independently selected from halogen, cyano, C₁-C₃ alkoxy, carbonyl, -CONHR¹³ and amino; preferably selected from halogen, -CONHR¹³ and cyano;
m is 0, 1, 2 or 3;
R¹² is selected from hydrogen, cyano, halogen, substituted or unsubstituted C₁-C₄ alkyl;
R¹³ is selected from hydrogen, substituted or unsubstituted C₁-C₆ alkyl, substituted or unsubstituted C₃-C₈ cycloalkyl, substituted or unsubstituted C₁-C₆ alkoxy, substituted or unsubstituted 3-8 membered heterocyclyl; preferably, R¹³ is selected from hydrogen, unsubstituted or halogen substituted C₁-C₄ alkyl, C₃-C₆ cycloalkyl, C₁-C₆ alkoxy;
wherein, the heterocyclyl refers to heterocyclic group containing 1-3 heteroatoms selected from N, O, and S; the substituted refers to being substituted with one or more selected from C₁-C₄ alkyl, C₁-C₆ alkoxy, halogen, hydroxyl, cyano, amino, carboxyl and C₃-C₆ cycloalkyl.

2. The compound, or the stereoisomer, the geometric isomer, the tautomer, the pharmaceutically acceptable salt or the hydrate thereof according to claim 1, wherein 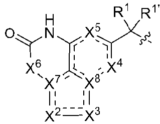 in formula (I) is selected from the following structures: 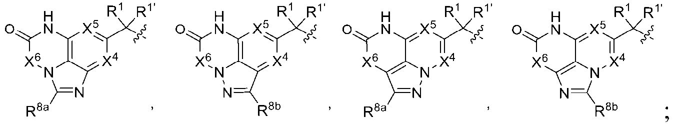 wherein, each substitution defined as said in claim 1.

3. The compound, or the stereoisomer, the geometric isomer, the tautomer, the pharmaceutically acceptable salt or the hydrate thereof according to claim 2, wherein 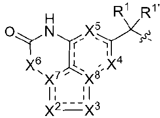 in formula (I) is selected from the following structures: 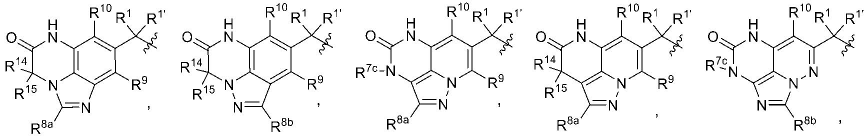 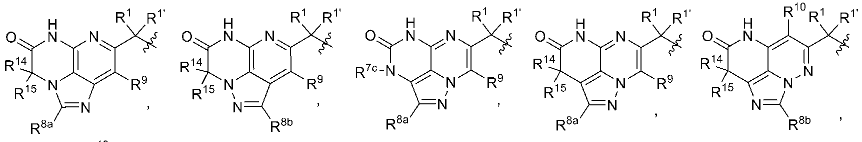 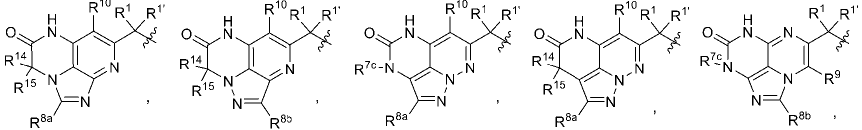 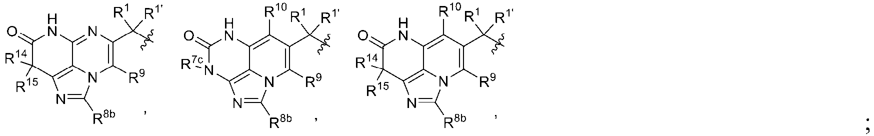 wherein, R^{8a} and R^{8b} are each independently selected from hydrogen, fluorine, chlorine, methyl, ethyl, cyano; R⁹ and R¹⁰ are each independently selected from hydrogen, fluorine, chlorine, methyl; each other substitutions defined as said in claim 1.

4. The compound, or the stereoisomer, the geometric isomer, the tautomer, the pharmaceutically acceptable salt or the hydrate thereof according to any claim of 1-3, wherein 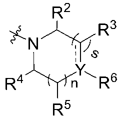 in formula (I) is selected from the following structures: 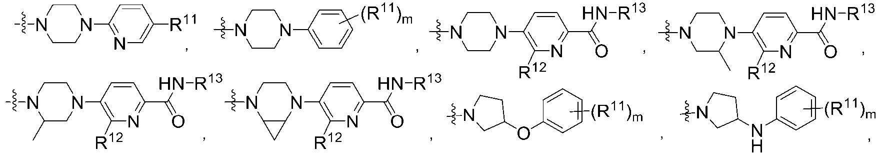 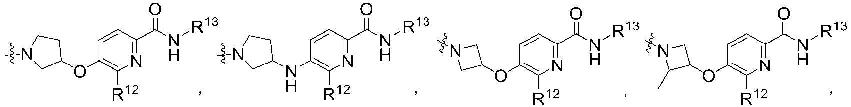 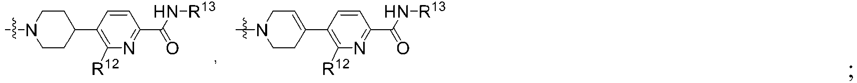 wherein, each substitution defined as said in claim 1.

5. The compound, or the stereoisomer, the geometric isomer, the tautomer, the pharmaceutically acceptable salt or the hydrate thereof according to claim 4, wherein 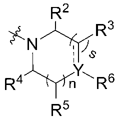 in formula (I) is selected from the following structures: 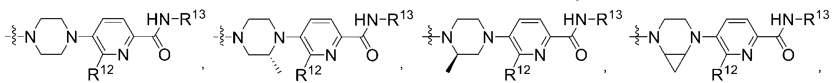 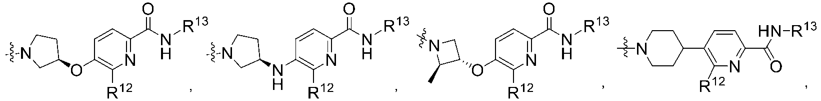 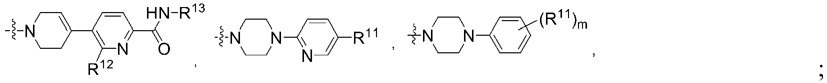 wherein, each substitution defined as said in claim 1.

6. The compound, or the stereoisomer, the geometric isomer, the tautomer, the pharmaceutically acceptable salt or the hydrate thereof according to any claim of 1-5, wherein, the compound of formula (I) is selected from the following compounds:
| | | |
|---|---|---|
| 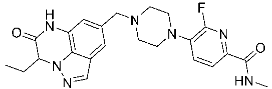 | 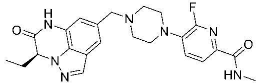 | 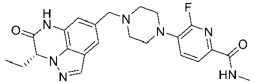 |
| 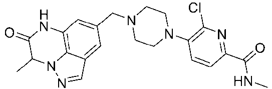 | 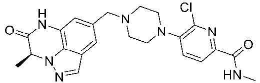 | 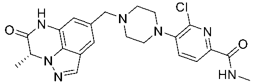 |
| 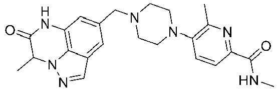 | 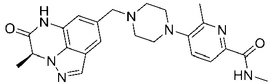 | 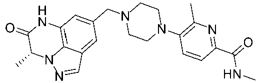 |
| 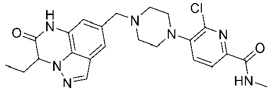 | 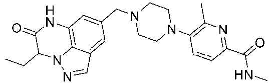 | 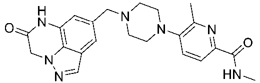 |
| 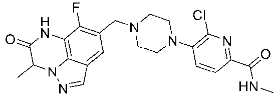 | 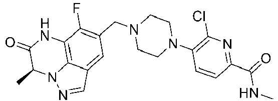 | 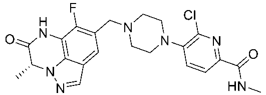 |
| 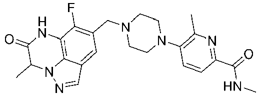 | 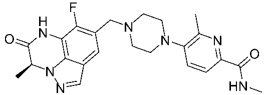 | 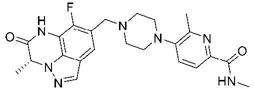 |
| 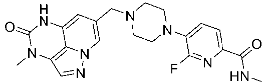 | 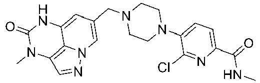 | 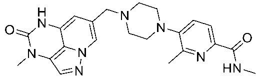 |
| 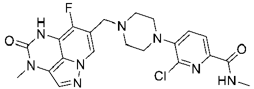 | 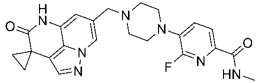 | 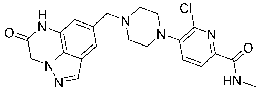 |
| 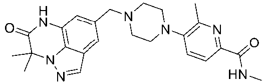 | 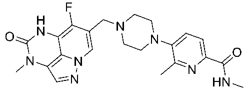 | 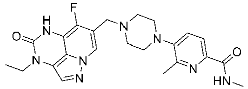 |
| 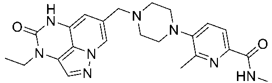 | 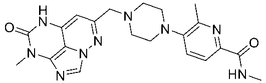 | 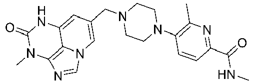 |
| 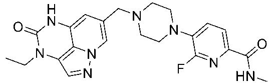 | 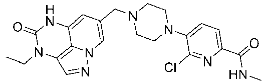 | 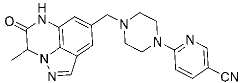 |
| 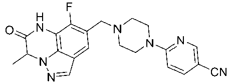 | 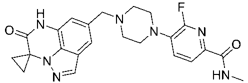 | 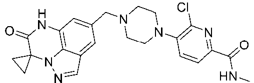 |
| 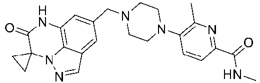 | 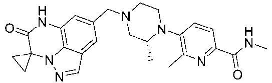 | 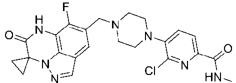 |
| 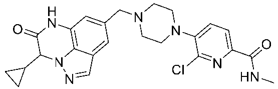 | 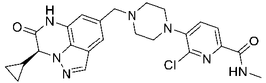 | 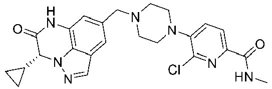 |
| 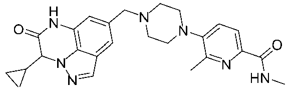 | 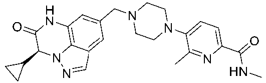 | 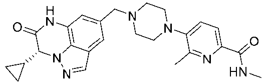 |
| 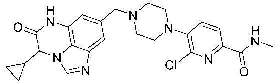 | 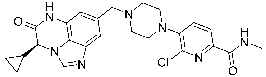 | 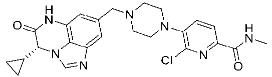 |
| 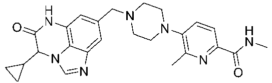 | 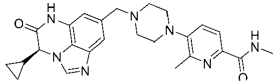 | 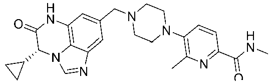 |
| 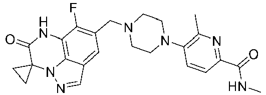 | 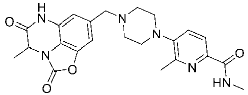 | 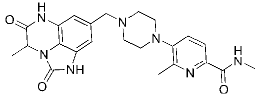 |
| 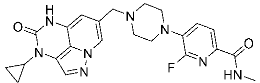 | 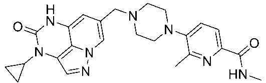 | 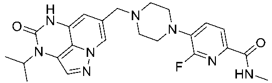 |
| 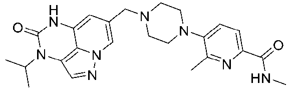 | 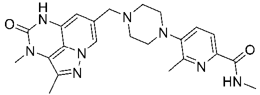 | 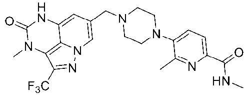 |
| 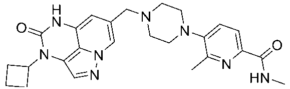 | 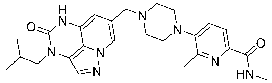 | 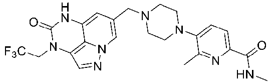 |
| 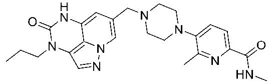 | 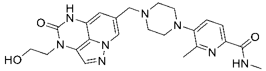 | 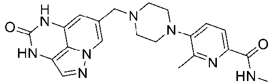 |
| 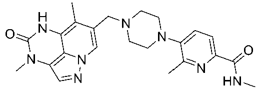 | 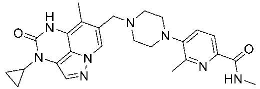 | 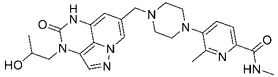 |
| 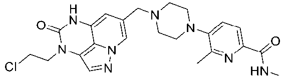 | 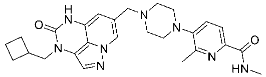 | 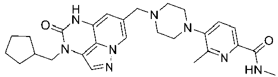 |
| 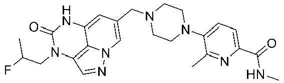 | 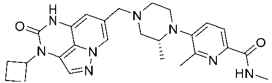 | 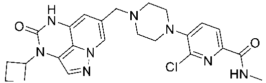 |
| 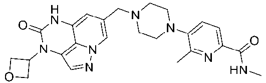 | 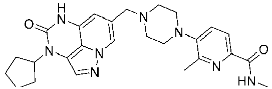 | 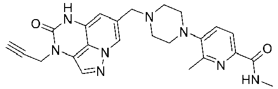 |
| 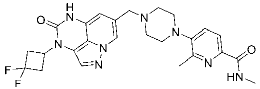 | 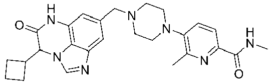 | 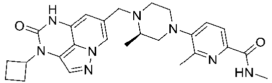 |
| 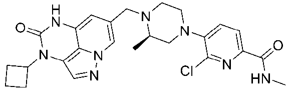 | 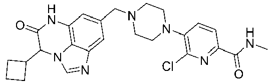 | 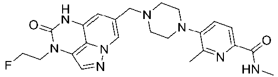 |

7. A pharmaceutical composition, comprising one or more selected from the compound, the stereoisomer, the geometric isomer, the tautomer, the pharmaceutically acceptable salt and the hydrate thereof according to any claim of 1-6, and an optional pharmaceutically acceptable carrier.

8. Use of the compound, or the stereoisomer, the geometric isomer, the tautomer, the pharmaceutically acceptable salt or the hydrate thereof according to any claim of 1-6, or the pharmaceutical composition according to claim 7 in the preparation of PARP1 inhibitor.

9. Use of the compound, or the stereoisomer, the geometric isomer, the tautomer, the pharmaceutically acceptable salt or the hydrate thereof according to any claim of 1-6, or the pharmaceutical composition according to claim 7 in the preparation of drugs for preventing and/or treating disease improved by inhibiting PARP1;
preferably, the disease improved by inhibiting PARP1 includes cancer.

10. The use according to claim 9, wherein, the cancer includes malignant tumors, such as any of ovarian cancer, breast cancer, fallopian tube cancer, endometrial cancer, peritoneal cancer, gastric cancer, colon cancer, bladder cancer, pancreatic cancer, biliary cancer, osteosarcoma, cervical cancer, head and neck tumors, germ cell and embryonic cancer, esophageal cancer, malignant glioma, Ewing sarcoma, pancreatic cancer, melanoma, bile duct cancer, prostate cancer, small cell lung cancer, non-small cell lung cancer, lymphoma and blood cancer;
preferably, the genome of the cancer is a type lacking homologous recombination repair;
preferably, the cancer is dependent on a pathway where double-strand DNA damage is repaired without homologous recombination;
preferably, the cancer comprises one or more cancer cells that lack the ability to repair double-strand DNA breaks through homologous recombination relative to normal cells;
preferably, the cancer comprises one or more cancer cells that lack BRCA1 or BRCA2, or that have BRCA1 or BRCA2 mutation.
